# EUROPEAN PATENT SPECIFICATION

(11) **EP 4 472 384 B1**
(45) Date of publication and mention of the grant of the patent: **19.11.2025**
(21) Application number: 23176114.9
(22) Date of filing: 30.05.2023
(51) Int. Cl.: H10K 85/40, H10K 85/60, C07D 251/24, C07F 7/08, H10K 50/165, H10K 50/16

(54) **COMPOUND, ORGANIC LIGHT EMITTING DIODE, AND DISPLAY DEVICE**
VERBINDUNG, ORGANISCHE LICHTEMITTIERENDE DIODE UND ANZEIGEVORRICHTUNG
COMPOSÉ, DIODE ÉLECTROLUMINESCENTE ORGANIQUE ET DISPOSITIF D'AFFICHAGE

(43) Date of publication of application: 04.12.2024
(73) Proprietor: Novaled GmbH, 01099 Dresden (DE)
(72) Inventor: GALÁN GARCÍA, Elena, 01099 Dresden (DE); KARPOV, Yevhen, 01099 Dresden (DE)
(74) Representative: Bittner, Thomas L.

(56) References cited:
- WO-A1-2022/239962
- CN-A- 111 072 637

## Description

### TECHNICAL FIELD

The present invention relates to a compound. The present invention relates further to an organic light emitting diode comprising the compound, to a display device comprising the organic light emitting diode, and to a process for preparing the organic light emitting diode.

### BACKGROUND OF THE INVENTION

Organic semiconducting devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic semiconducting device, such as an organic light emitting diode, may be applied to a large-size flat panel display.

It is, therefore, the object of the present invention to provide organic light emitting diodes and compounds for preparing the same overcoming drawbacks of the prior art, in particular providing compounds for use in organic light emitting diodes comprising the same helpful to improve the performance thereof, especially with respect to efficiency.

WO 2022/239962 A1 discloses a compound represented by chemical formula the following chemical formula 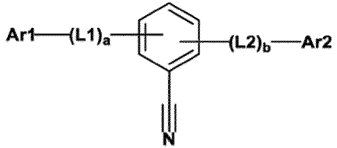 and an organic light-emitting device comprising same.

### DISCLOSURE

This object is achieved by a compound of the following formula (I) 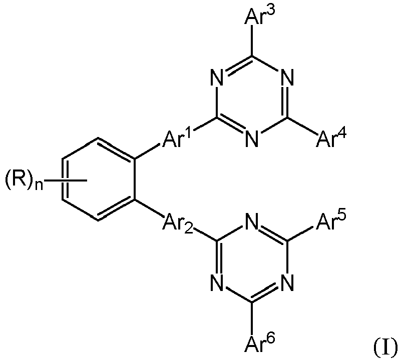 wherein
n is an integer from 1 to 4;
R is independently selected from the group consisting of C₁ to C₁₂ alkyl, partially fluorinated C₁ to C₁₂ alkyl, perfluorinated C₁ to C₁₂ alkyl, F, and SiR'₃, wherein R' is independently selected from C₁ to C₁₂ alkyl;
Ar¹ and Ar² are independently selected C₆ to C₁₂ arylene; and
Ar³ to Ar⁶ are independently selected C₆ to C₁₂ aryl.

The object is further achieved by an organic light emitting diode comprising a substrate, an anode, a cathode, an emission layer, and a first electron transport layer,
wherein
- the first electron transport layer is arranged between the emission layer and the cathode; and
- the first electron transport layer comprises the compound of formula (I) according to the present invention.

This object is further achieved by a display device comprising the organic light emitting diode according to the present invention.

This object is further achieved by a process for preparing the organic light emitting diode according to the invention, wherein the process comprises a step of depositing the compound according to the present invention on a solid support.

Surprisingly, it was found that the compound according to the invention can be used for preparing organic light emitting diodes and display devices having improved properties in comparison with respective devices of the prior art, especially with improved (higher) current efficiency at comparable operating voltage and lifetime.

### Compound

According to one aspect, the invention is related to a compound of formula (I)

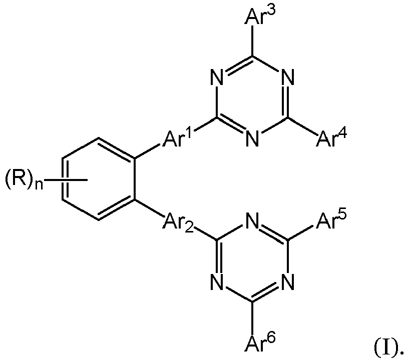

If not mentioned else explicitly, all compounds, especially the compound of formula (I), groups, moieties, substituents etc. shown herein, especially by structural formulas, by systematic names etc. encompass the respective partially and fully deuterated derivatives thereof and/or derivatives substituted with one or more C₁ to C₄ alkyl groups.

Especially, if not mentioned else explicitly, all compounds, especially the compound of formula (I), groups, moieties, substituents etc. shown herein, especially by structural formulas, by systematic names etc. encompass the respective partially and fully deuterated derivatives thereof, wherein it may be provided that substitution beyond deuteration is excluded.

In accordance with the present disclosure, as an illustrative example using an arbitrary group A in a formula showing the following binding situation, 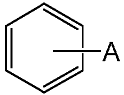 the group A may be bound to any suitable binding position. That is, in formula (I) the one or more R may be bonded to the positions marked with the *-symbol in the following formula, respectively

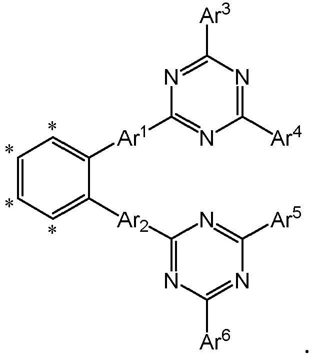

In formula (I), n is an integer from 1 to 4. n may be an integer selected from the group consisting of 1, 2, and 4. n may be 1 or 2. n may be 1.

The compound of formula may have R-groups at the positions market with the *-symbol in the following formulas I-1 to I-6

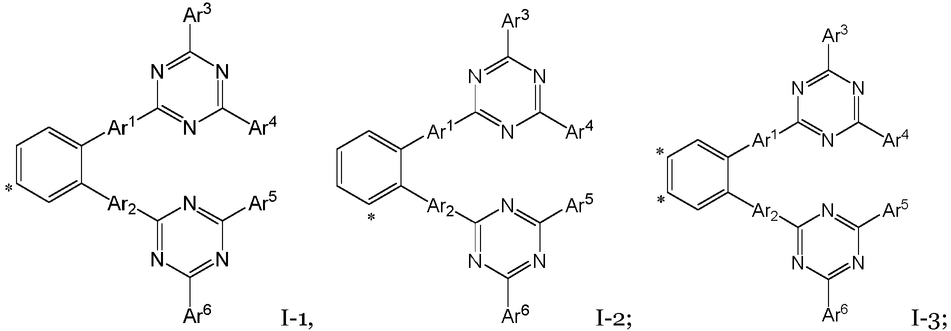

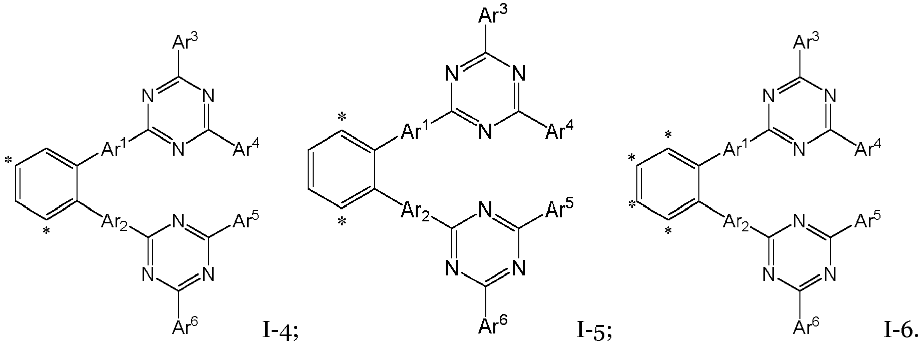

The compound of formula may have R-groups at the positions market with the *-symbol in formulas I-1 and I-3.
Ar¹ and Ar² are independently selected C₆ to C₁₂ arylene. Ar¹ and Ar² may be independently selected from the group consisting of phenylene, biphenyl-ene and naphtylene, that is, divalent groups of the following formulas 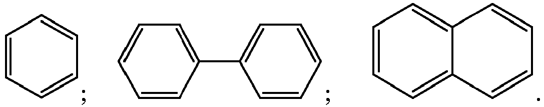
Ar¹ and Ar² may be independently selected from the group consisting of 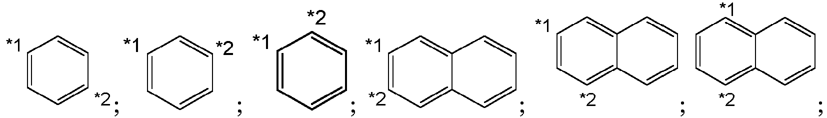 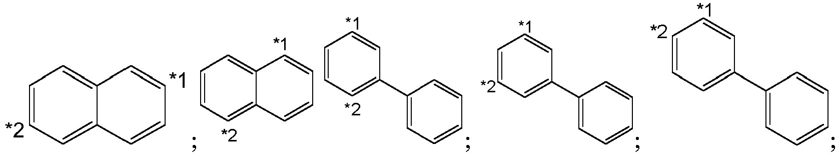 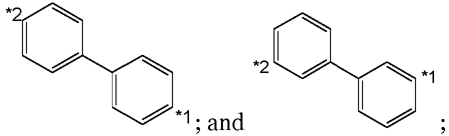 wherein the respective group is bonded at *1 to 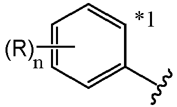 in formula (I), respectively; and
the respective group is bonded at *2 to 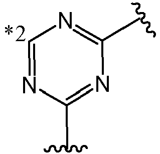 in formula (I), respectively.
Ar¹ and Ar² may be both phenylene. Ar¹ and Ar² may be independently selected from the group consisting of 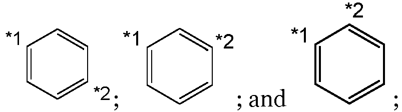 wherein the respective group is bonded at *1 to 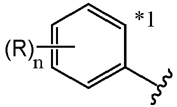 in formula (I), respectively; and
the respective group is bonded at *2 to 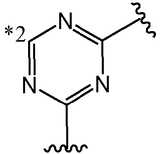 in formula (I), respectively.
Ar¹ and Ar² may be independently selected from the group consisting of 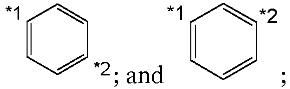 wherein the respective group is bonded at *1 to 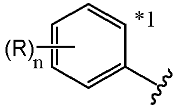 in formula (I), respectively; and
the respective group is bonded at *2 to 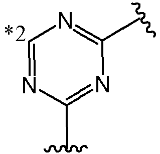 in formula (I), respectively.
Ar³ to Ar⁶ are independently selected C₆ to C₁₂ aryl. Ar³ to Ar⁶ may be independently selected from the group consisting of phenyl, biphenyl and naphtyl, that is, monovalent groups of the following formulas 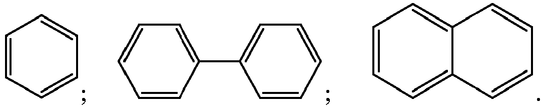
Ar³ to Ar⁶ may be independently selected from the group consisting of 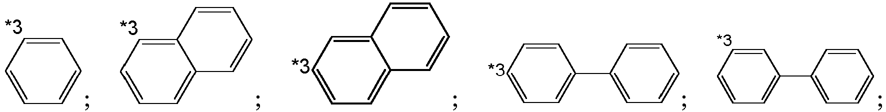 and 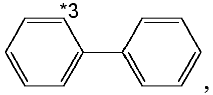 wherein
the respective group is bonded at *3 to 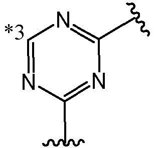 in formula (I), respectively.
Ar³ to Ar⁶ may be each phenyl.

The compound of formula (I) may have the formula (Ib) 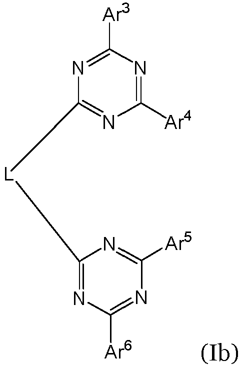
wherein L is independently selected from L-1 to L-5 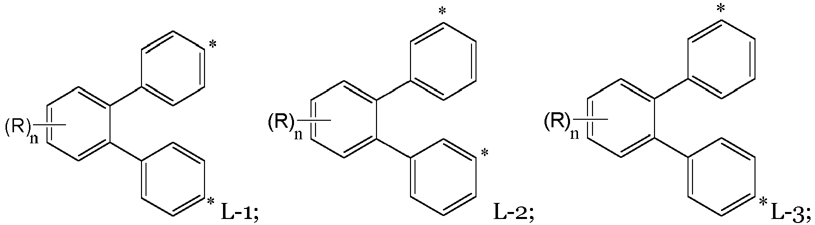 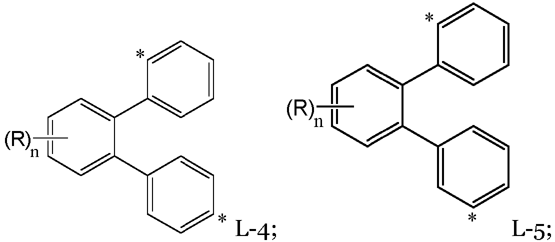
wherein the * represent the binding positions, that is, the positions at which 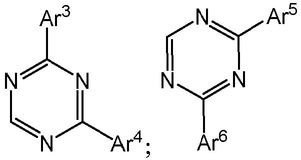
are bonded, respectively.

In formula Ib, L may be selected from L-1 to L-4. In formula Ib, L may be selected from L-1 to L-3.

The compound of formula (I) may have the formula (Ia) 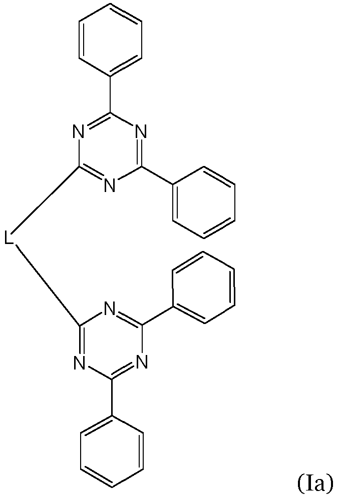
wherein L is independently selected from L-1 to L-5 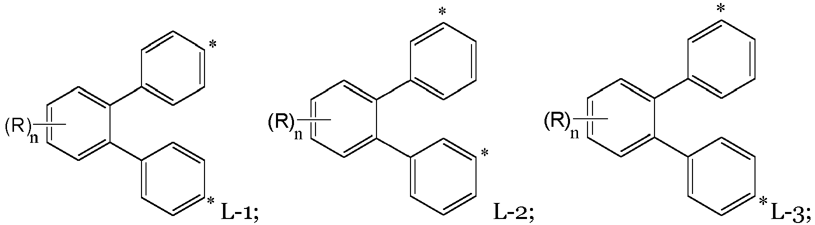 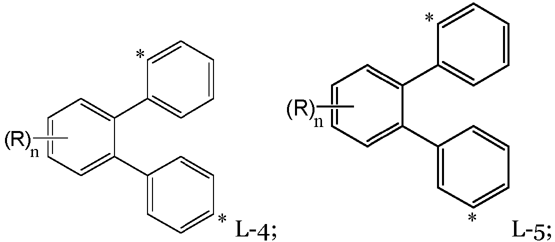
wherein the * represent the binding positions that is, the positions at which 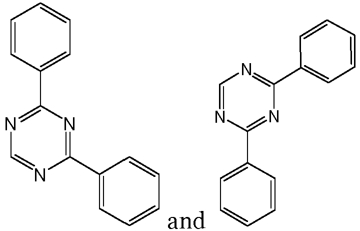
are bonded, respectively.

In formula Ia, L may be selected from L-1 to L-4. In formula Ia, L may be selected from L-1 to L-3.

R is independently selected from the group consisting of C₁ to C₁₂ alkyl, partially fluorinated C₁ to C₁₂ alkyl, perfluorinated C₁ to C₁₂ alkyl, F, and SiR'₃, wherein R' is independently selected from C₁ to C₁₂ alkyl. R may be independently selected from the group consisting of C₁ to C₁₀ alkyl, partially fluorinated C₁ to C₁₀ alkyl, perfluorinated C₁ to C₁₀ alkyl, F, and SiR'₃, wherein R' is independently selected from C₁ to C₁₀ alkyl. R may be independently selected from the group consisting of C₁ to C₈ alkyl, partially fluorinated C₁ to C₈ alkyl, perfluorinated C₁ to C₈ alkyl, F, and SiR'₃, wherein R' is independently selected from C₁ to C₈ alkyl. R may be independently selected from the group consisting of C₁ to C₆ alkyl, partially fluorinated C₁ to C₆ alkyl, perfluorinated C₁ to C₆ alkyl, F, and SiR'₃, wherein R' is independently selected from C₁ to C₆ alkyl. R may be independently selected from the group consisting of C₁ to C₄ alkyl, partially fluorinated C₁ to C₄ alkyl, perfluorinated C₁ to C₄ alkyl, F, and SiR'₃, wherein R' is independently selected from C₁ to C₄ alkyl.

R may be independently selected from the group consisting of R-1 to R-6

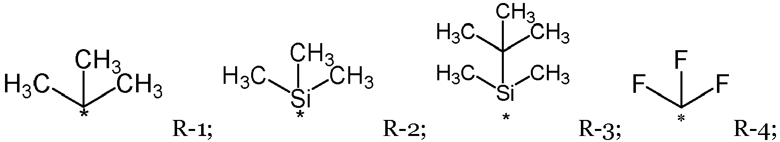

F * _{R-5}; * CH_{3_{R-6}};

wherein the * represent the binding position, that is, the binding position to one of the positions marked with a *-symbol in the following formula

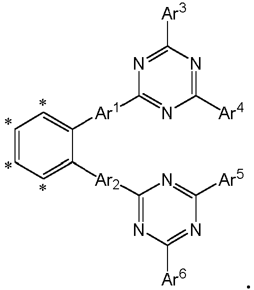

R may be independently selected from the group consisting of R-1, R-2, R-4 and R-5

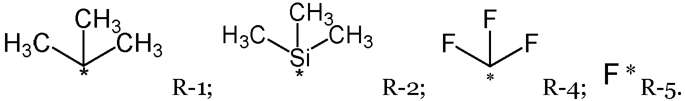

In one embodiment, the compound in accordance with the present invention has the formula (I) 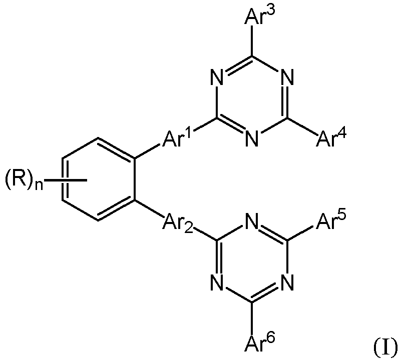 wherein
n is 1 or 2;
Ar¹ and Ar² are each phenylene;
Ar³ to Ar⁶ are each phenyl; and
R is independently selected from the group consisting of C₁ to C₄ alkyl, partially fluorinated C₁ to C₄ alkyl, perfluorinated C₁ to C₄ alkyl, F, and SiR'₃, wherein R' is independently selected from C₁ to C₄ alkyl.

In one embodiment, the compound in accordance with the present invention has the formula (Ia) 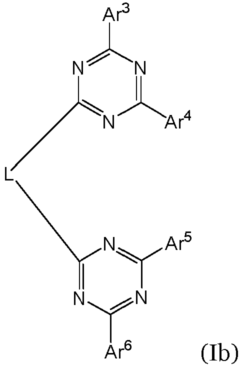 wherein
L is independently selected from L-1 to L-5 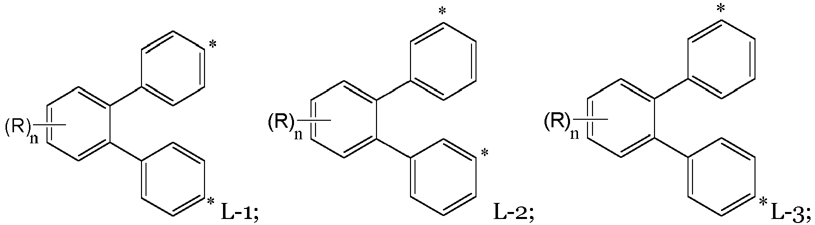 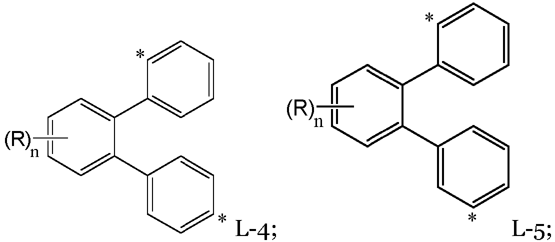
wherein the * represent the binding positions, that is, the positions at which 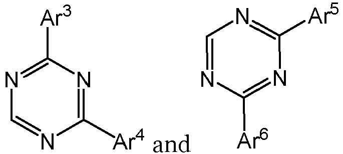
are bonded, respectively;
n is 1 or 2;
Ar¹ and Ar² are each phenylene;
Ar³ to Ar⁶ are each phenyl; and
R is independently selected from the group consisting of C₁ to C₄ alkyl, partially fluorinated C₁ to C₄ alkyl, perfluorinated C₁ to C₄ alkyl, F, and SiR'₃, wherein R' is independently selected from C₁ to C₄ alkyl.

In one embodiment, the compound in accordance with the present invention has the formula (Ia) 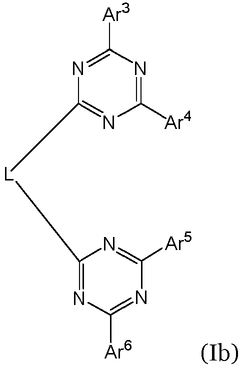 wherein
L is independently selected from L-1 to L-5 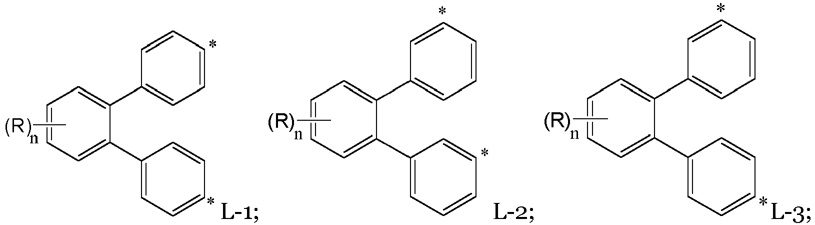 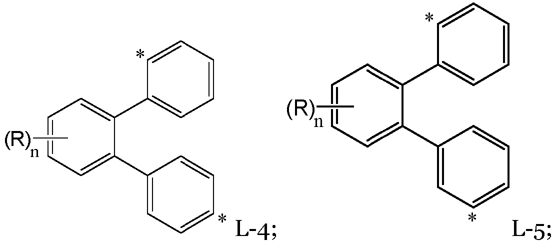
wherein the * represent the binding positions, that is, the positions at which 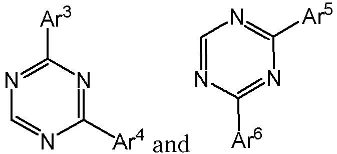
are bonded, respectively;
n is 1 or 2;
Ar¹ and Ar² are independently selected from meta-phenylene and para-phenylene;
Ar³ to Ar⁶ are each phenyl; and
R is independently selected from the group consisting of R-1, R-2, R-4 and R-5

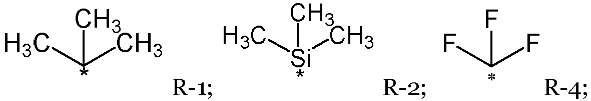

   F * _{R-5};
wherein the * represent the binding position, that is, the binding position to one of the positions marked with a *-symbol in the following formula

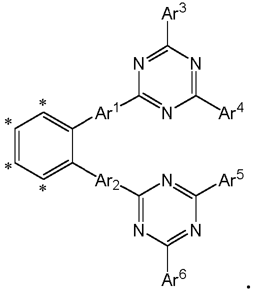

The compound of formula (I) in accordance with the present invention may have a HOMO energy level from -6.40 eV to -5.80 eV, from -6.30 eV to -5.90 eV, or from -6.29 eV to -5.92 eV.

The compound of formula (I) in accordance with the present invention may have a LUMO energy level from -2.00 eV to -1.70 eV, from -1.95 eV to -1.75 eV, or from -1.92 eV to -1.79 eV.

The compound of formula (I) in accordance with the present invention may have an E-gap between the HOMO and the LUMO (energy difference between HOMO and LUMO) from 3.90 eV to 4.50 eV, from 4.00 eV to 4.40 eV, or from 4.02 to 4.38 eV.

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

The compound of formula (I) in accordance with the present invention may have a dipole moment from 0.30 Debye to 3.50 Debye, from 0.50 Debye to 3.00 Debye, or from 0.52 Debye to 2.93 Debye.

The dipole moment |*̅µ̅*̅| of a molecule containing N atoms is given by: 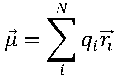 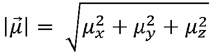 where *qᵢ*and *̅r̅ᵢ̅*̅ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

The compound of formula (I) in accordance with the present invention may have a melting point from 270 °C to 345°C, from 280°C to 342°C, or from 281°C to 341°C.

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 µL Mettler Toledo aluminum pan with lid, a <1 mm hole is pierced into the lid).

The compound of formula (I) in accordance with the present invention may have a glass transition temperature (Tg) from 110°C to 165°C, from 120°C to 160°C, or from 123°C to 155°C.

The glass transition temperature (Tg) is measured under nitrogen and using a heating rate of 10 K per min in a Mettler Toledo DSC 822e differential scanning calorimeter as described in DIN EN ISO 11357, published in March 2010.

The compound of formula (I) in accordance with the present invention may have a rate onset temperature from 230°C to 280°C, from 240°C to 270°C, or from 243°C to 268°C.

The rate onset temperature (TRO) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10-5 mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

The compound of formula (I) in accordance with the present invention may have a reduction potential from -2.3 V to -2.0 V, from -2-2 V to -2.1 V, or from -2.19 V to -2.13 V.

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1 M concentration) as the standard. The average of potentials corresponding to cathodic and anodic peak of the studied compound, after subtraction of the average of cathodic and anodic potentials observed for the standard Fc⁺/Fc redox couple, afforded finally the values reported above. All studied compounds as well as the reported comparative compounds showed well-defined reversible electrochemical behaviour.

The compound of formula (I) may comprise 8 to 13 aromatic or heteroaromatic rings, 8 to 11 aromatic or heteroaromatic rings, 9 to 11 aromatic or heteroaromatic rings, or may comprise 9 aromatic or heteroaromatic rings. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)aromatic rings, each ring is considered as a single ring in this regard. For example, naphthalene comprises two aromatic rings.

The compound of formula (I) may comprise 2 to 4 heteroaromatic rings, 2 to 3 heteroaromatic rings, or 2 heteroaromatic rings.

The aromatic or heteroaromatic rings of the electron transport compound may be 6-membered rings.

The heteroaromatic rings may be N-containing heteroaromatic rings, optionally all of the heteroaromatic rings are N-containing heteroaromatic rings, optionally all of the heteroaromatic rings heteroaromatic rings contain N as the only type of heteroatom.

At least one six-member heteroaromatic ring may contain one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the electron transport compound may be triazine, diazine, pyrazine, pyrimidine, pyridine, quinazoline or benzoquinazoline, preferably triazine.

If the electron transport compound comprises two or more heteroaromatic rings, the heteroaromatic rings may be separated from each other by at least one aromatic ring which is free of a heteroatom.

The compound of formula (I) may have a molecular weight from 650 g/mol to 2,500 g/mol, from 680 g/mol to 2,000 g/mol, from 700 g/mol to 1,800 g/mol, from 706 g/mol to 1,650 g/mol.

The compound of formula (I) according to the invention may be selected from E-1 to E-28

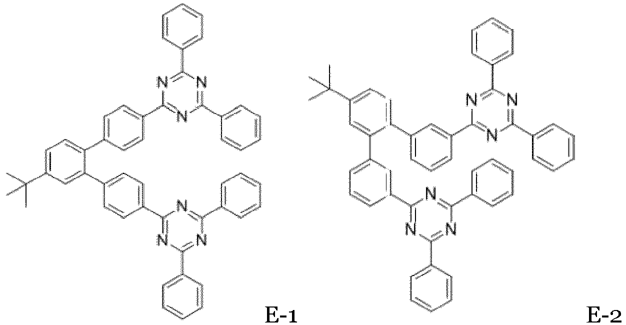

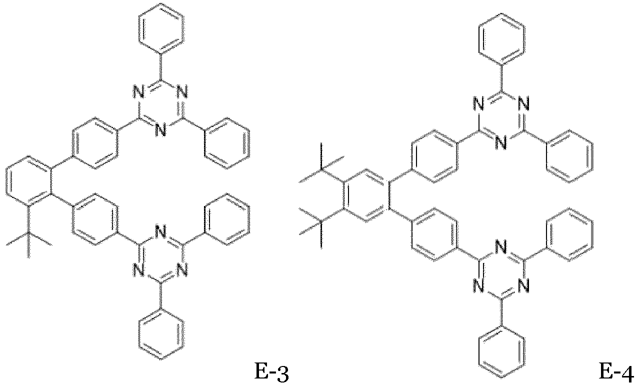

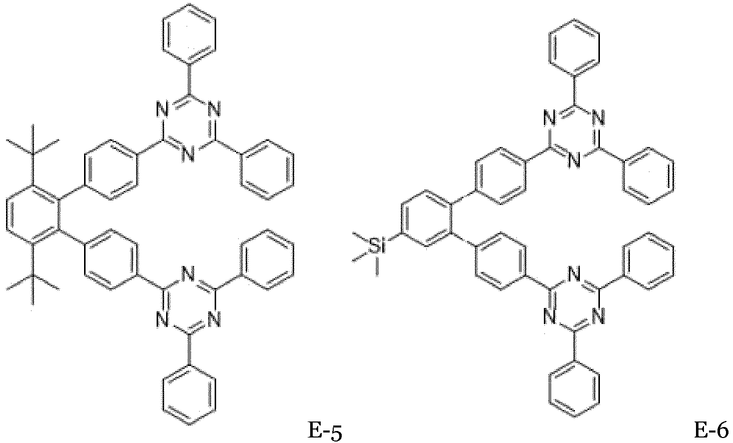

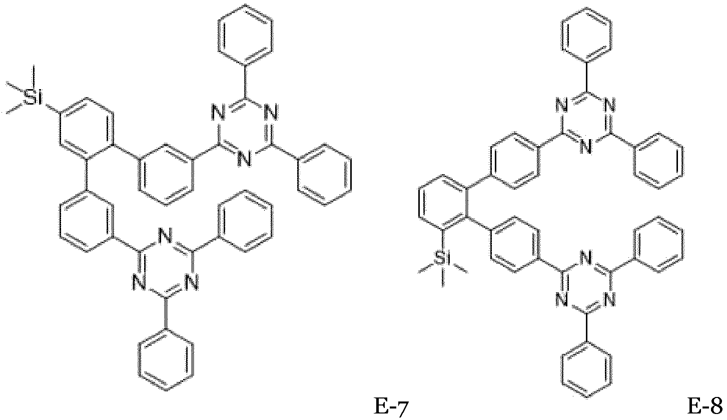

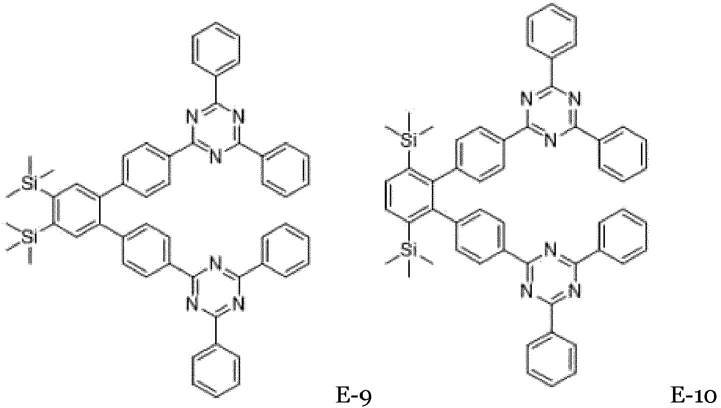

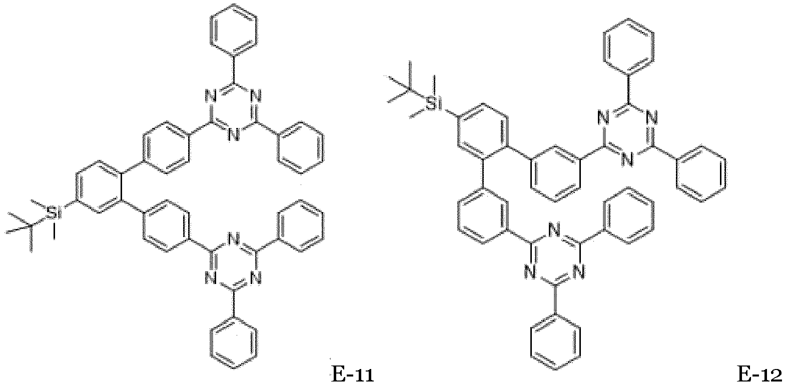

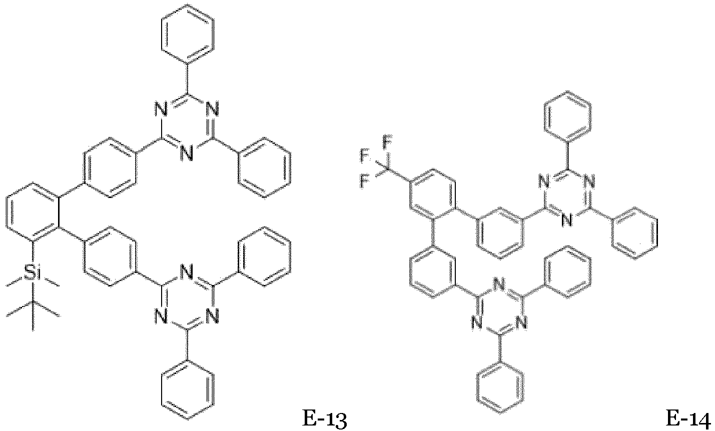

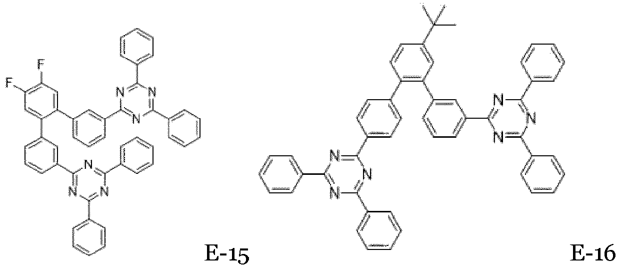

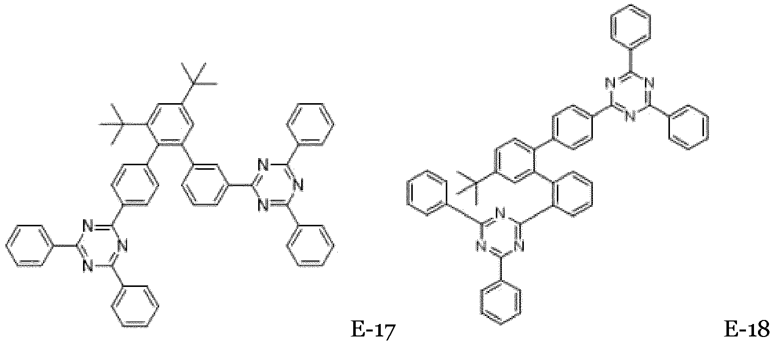

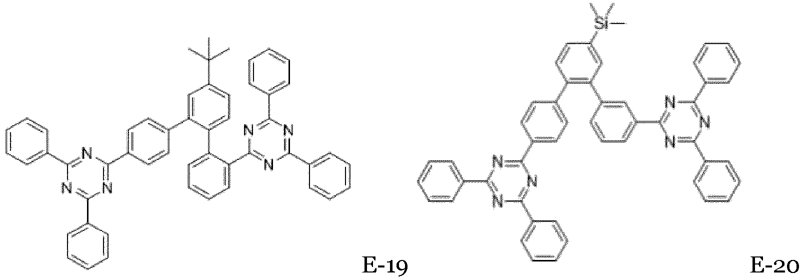

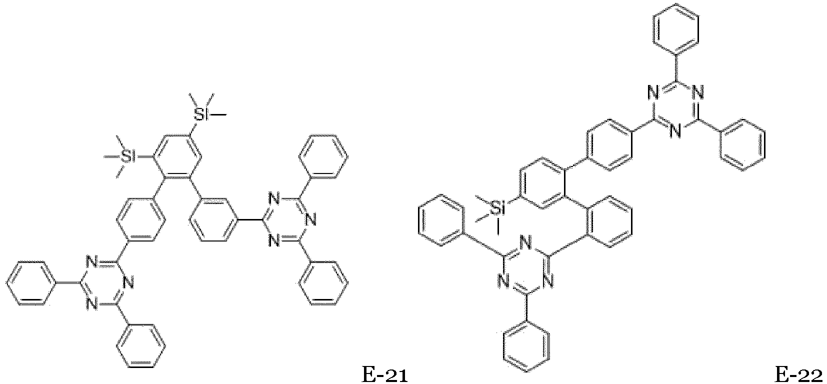

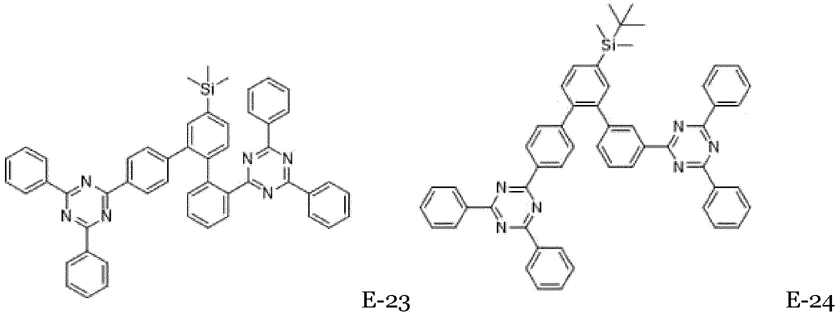

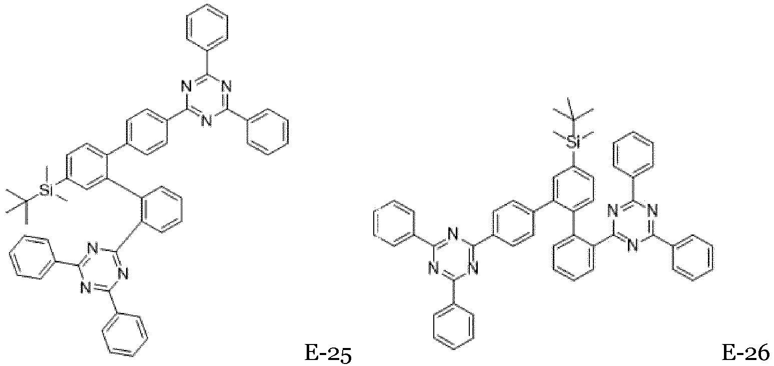

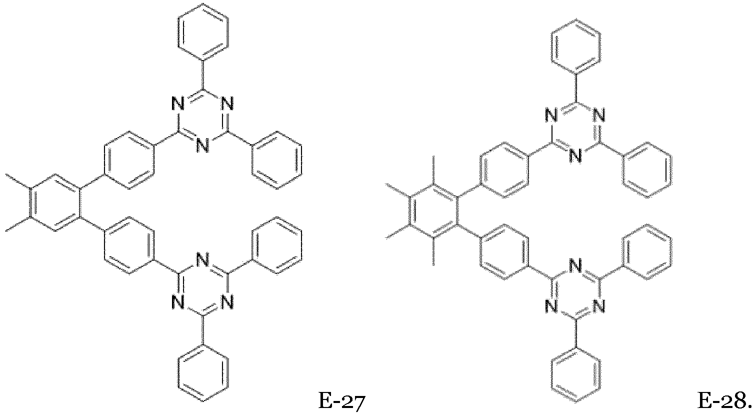

### Organic light emitting diode

The object is further achieved by an organic light emitting diode comprising a substrate, an anode, a cathode, an emission layer, and a first electron transport layer,
wherein
- the first electron transport layer is arranged between the emission layer and the cathode; and
- the first electron transport layer comprises the compound of formula (I) according to the present invention.

The organic light emitting diode is a single stack organic light emitting diode or a multi-stack organic light emitting diode.

The first electron transport layer may comprise the compound of formula (I) in accordance with the invention in an amount of at least 50 wt.-% with respect to the total weight of the first electron transport layer. The first electron transport layer may comprise the compound of formula (I)in accordance with the invention in an amount of at least 60 wt.-% with respect to the total weight of the first electron transport layer. The first electron transport layer may comprise the compound of formula (I) in accordance with the invention in an amount of at least 70 wt.-% with respect to the total weight of the first electron transport layer. The first electron transport layer may comprise the compound of formula (I) in accordance with the invention in an amount of at least 80 wt.-% with respect to the total weight of the first electron transport layer. The first electron transport layer may comprise the compound of formula (I) in accordance with the invention in an amount of at least 90 wt.-% with respect to the total weight of the first electron transport layer. The first electron transport layer may comprise the compound of formula (I) in accordance with the invention in an amount of at least 95 wt.-% with respect to the total weight of the first electron transport layer. The first electron transport layer may comprise the compound of formula (I) in accordance with the invention in an amount of at least 98 wt.-% with respect to the total weight of the first electron transport layer. The first electron transport layer may comprise the compound of formula (I) in accordance with the invention in an amount of at least 99 wt.-% with respect to the total weight of the first electron transport layer. The first electron transport layer may substantially consist of the compound of formula (I) in accordance with the invention. The first electron transport layer may consist of the compound of formula (I) in accordance with the invention.

The first electron transport layer may have a thickness from 10 to 50 nm, from 20 to 40 nm, or from 25 to 35 nm, such as about 30 nm, for example 31 nm.

### Electrical dopant

The first electron transport layer may comprise, in addition to the compound of formula (I) in accordance with the invention an electrical dopant, such as an n-type dopant, especially a redox n-type dopant.

Under electrical dopant, especially n-type dopant it is understood a compound which, if embedded into an electron transport matrix, improves, in comparison with the neat matrix under the same physical conditions, the electron properties of the formed organic material, particularly in terms of electron injection and/or electron conductivity.

In the context of the present invention "embedded into an electron transport matrix" means homogenously mixed with the electron transport matrix.

The electrical dopant as referred to herein is especially selected from elemental metals, metal salts, metal complexes and organic radicals.

In one embodiment, the electrical dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium complex has the formula II, III or IV: 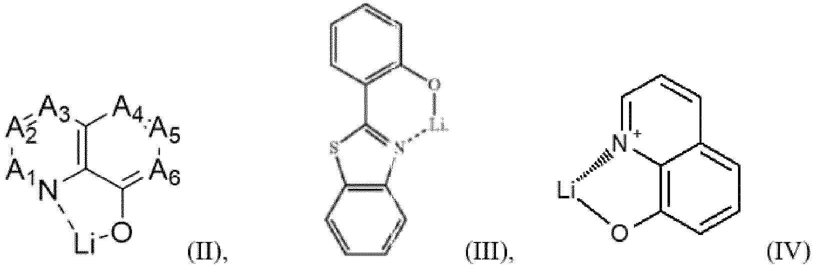 wherein
   A₁ to A₆ are same or independently selected from CH, CR, N, O;
   R is same or independently selected from hydrogen, halogen, alkyl or aryl or heteroaryl with 1 to 20 carbon atoms; and more preferred A₁ to A₆ are CH,

   - the borate based organic ligand is a tetra(1H-pyrazol-1-yl)borate,
   - the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
   - the pyridinolate is a 2-(diphenylphosphoryl)pyridin-3-olate,
- the lithium Schiff base has the structure 100, 101, 102 or 103:

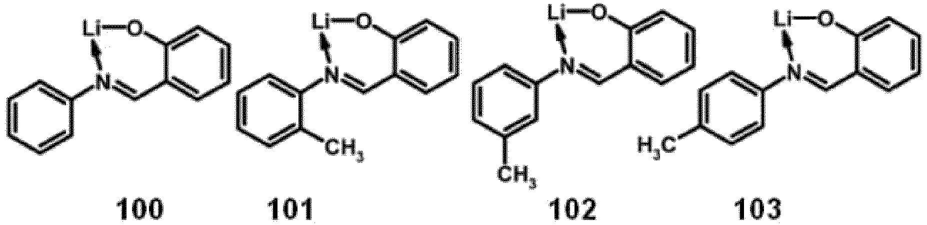

According to one embodiment of the invention, the first electron transport layer of the present invention comprises a lithium organic complex, especially may comprise 8-hydroxyquinolinolato-lithium (= LiQ).

According to one embodiment of the present invention the first electron transport layer comprsises a metal, preferably selected from alkali metals, alkaline earth metals, rare earth metals and metals of the first transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-type dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound.

In case that the n-type dopant is an electrically neutral metal complex and/or an electrically neutral organic radical, the measurement of its redox potential is actually performed for the redox couple formed by
(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of
(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.

Electrically neutral metal complexes suitable as n-type dopants may be e.g. strongly reductive complexes of some transition metals in low oxidation state. Particularly strong n-type dopants may be selected for example from Cr(II), Mo(II) and/or W(II) guanidinate complexes such as W₂(hpp)₄, as described in more detail in WO2005/086251.

Electrically neutral organic radicals suitable as n-type dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form. It is further understood that if the vaporized elemental metal is deposited together with a covalent matrix, the metal atoms and/or clusters are embedded in the covalent matrix. In other words, it is understood that any metal-doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the electrical may be selected from electropositive metals selected from alkali metals, alkaline earth metals, rare earth metals and metals of the first transition period Ti, V, Cr and Mn. Preferably, the n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

### Auxiliary electron transport layer

The organic light emitting diode may further comprise an auxiliary electron transport layer, wherein the auxiliary electron transport layer is arranged between the emission layer and the first electron transport layer.

The auxiliary electron transport layer may also be referred to as a second electron transport layer or as a hole blocking layer.

The auxiliary electron transport layer may be in direct contact with the first electron transport layer. The auxiliary electron transport layer may be in direct contact with the emission layer. The auxiliary electron transport layer may be contacting sandwiched between the emission layer and the first electron transport layer.

The auxiliary electron transport layer may have a thickness of < 50 nm, optionally between 1 and 30 nm, optionally between 1 and 10 nm, optionally between 1 and 5 nm.

The auxiliary electron transport layer may comprise or consist of a compound (III), wherein the compound (III) comprises 8 to 13 aromatic or heteroaromatic rings, optionally 8 to 11 aromatic or heteroaromatic rings, optionally 8 to 10 aromatic or heteroaromatic rings, and optionally 8 aromatic or heteroaromatic rings, wherein one or more of the aromatic or heteroaromatic rings may be substituted with C₁ to C₄ alkyl. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)aromatic rings, each ring is considered as a single ring in this regard. For example, naphthalene comprises two aromatic rings.

The compound (III) may comprise at least one heteroaromatic ring, optionally 1 to 5 heteroaromatic rings, optionally 1 to 4 heteroaromatic rings, optionally 1 to 3 heteroaromatic rings, and optionally 1 or 2 heteroaromatic rings.

The aromatic or heteroaromatic rings of the compound (III) may be 6-membered rings.

The heteroaromatic rings of the compound (III) may be a N-containing heteroaromatic ring, optionally all of the heteroaromatic rings are N-containing heteroaromatic rings, optionally all of the heteroaromatic rings heteroaromatic rings contain N as the only type of heteroatom.

The compound (III) may comprise at least one 6-membered heteroaromatic ring containing one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the compound (III) may be an azine. The at least one 6-membered heteroaromatic ring comprised in the compound (III) may be triazine, diazine, such as pyrimidine, or pyrazine.

If the compound (III) comprises two or more heteroaromatic rings, the heteroaromatic rings may be separated from each other by at least one aromatic ring which is free of a heteroatom.

For example, the auxiliary electron transport layer may comprise or consist of the following compound

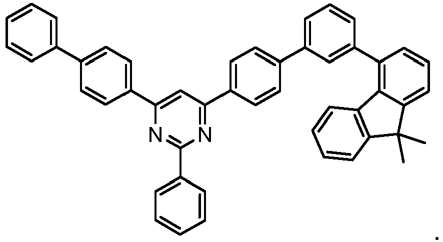

### Electron injection layer

The organic light emitting diode may further comprise an electron injection layer, wherein the electron injection layer is arranged between the first electron transport layer and the cathode. The electron injection layer may be in direct contact with the first electron transport layer. The electron injection layer may be in direct contact with the cathode. The electron injection layer may be contacting sandwiched between the first electron transport layer and the cathode.

The first electron transport layer may be contacting sandwiched between the auxiliary electron transport layer and the electron injection layer.

It may be provided that the electron injection layer does not comprise the compound of formula (I).

The electron injection layer may comprise a metal salt or a metal complex, preferably a lithium salt or a lithium organic complex; more preferably a compound selected from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium complex has the formula II, III or IV: 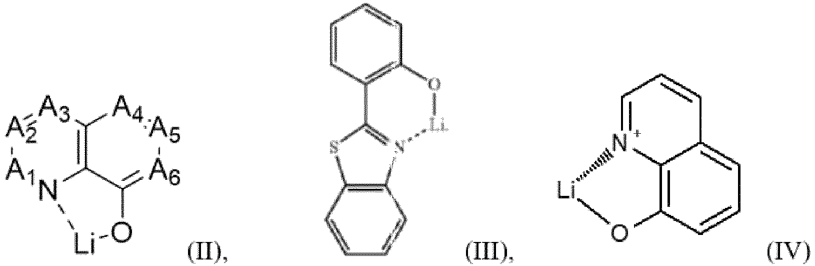 wherein
   A₁ to A₆ are same or independently selected from CH, CR, N, O;
   R is same or independently selected from hydrogen, halogen, alkyl or aryl or heteroaryl with 1 to 20 carbon atoms; and more preferred A₁ to A₆ are CH,

   - the borate based organic ligand is a tetra(1H-pyrazol-1-yl)borate,
   - the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
   - the pyridinolate is a 2-(diphenylphosphoryl)pyridin-3-olate,
- the lithium Schiff base has the structure 100, 101, 102 or 103:

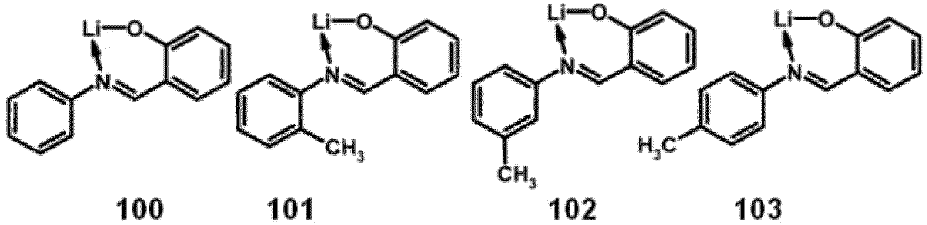

The electron injection layer may comprise or consist of a metal selected form the group consisting of alkali metals, alkaline earth metals, and rare earth metals, preferably the metal may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb most preferred Yb.

The thickness of the electron injection layer may be in the range from about 0.1 nm to about 10 nm, for example, in the range from about 0.5 nm to about 5 nm. When the thickness of the EIL is within this range, the EIL may have satisfactory electron-injecting properties, without a substantial penalty in driving voltage.

The first electron injection sub-layer may be in direct contact with the electron transport layer and the first electron injection sub-layer may comprise a metal salt or a metal complex, preferably a lithium salt or a lithium organic complex; more preferably a compound selected from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium complex has the formula II, III or IV: 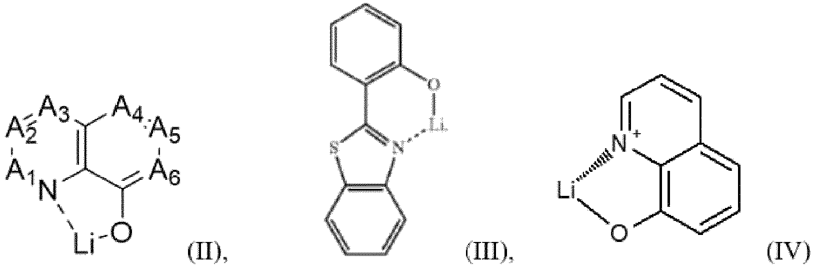 wherein
   A₁ to A₆ are same or independently selected from CH, CR, N, O;
   R is same or independently selected from hydrogen, halogen, alkyl or aryl or heteroaryl with 1 to 20 carbon atoms; and more preferred A₁ to A₆ are CH,

   - the borate based organic ligand is a tetra(1H-pyrazol-1-yl)borate,
   - the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
   - the pyridinolate is a 2-(diphenylphosphoryl)pyridin-3-olate,
- the lithium Schiff base has the structure 100, 101, 102 or 103:

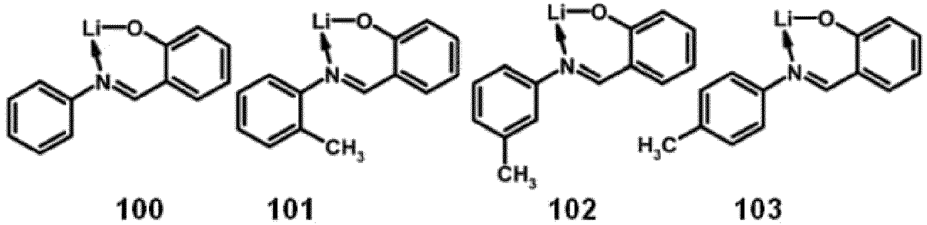

Preferably, the first electron injection sub-layer comprises 8-hydroxyquinolinolato-lithium ( = LiQ).

The second electron injection sub-layer comprises a metal selected form the group consisting of alkali metals, alkaline earth metals, and rare earth metals, preferably the metal may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb most preferred Yb.

The second electron injection sub-layer may be in direct contact with the cathode.

### Further layers

In accordance with the invention, the organic light emitting diode may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate. It may be provided that the substrate is a non-transparent substrate.

### Anode electrode

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO₂), aluminum zinc oxide (AlZO) and zinc oxide (ZnO), may be used to form the anode electrode. The anode electrode may also be formed using metals, typically silver (Ag), gold (Au), or metal alloys.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500°C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F₄TCNQ), 2,2'-(perfluoronaphthalen-2,6-diylidene) dimalononitrile or 2,2'2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F₄TCNQ), which LUMO level is about -5.2 eV; zinc phthalocyanine (ZnPc) (HOMO = -5.2 eV) doped with F₄TCNQ; α-NPD (N,N'-Bis(naphthalen-1-yl)-N,N'-bis(phenyl)-benzidine) doped with F₄TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL. The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Emission layer (EML)

The emission layer in the organic light emitting diode in accordance with the invention may be a blue emission layer or a green emission layer.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (II) or (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)₂).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)₃, and Btp₂lr(acac), but are not limited thereto. These compounds are phosphorescent emitters; however, fluorescent red emitter dopants could also be used.

Examples of phosphorescent green emitter dopants are Ir(ppy)₃ (ppy = phenylpyridine), Ir(ppy)₂(acac), Ir(mpyp)₃.

Examples of phosphorescent blue emitter dopants are F₂Irpic, (F₂ppy)₂Ir(tmd) and Ir(dfppz)₃ and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

The hole blocking layer may be the auxiliary electron transport layer as defined above. The hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as α-ETL or auxiliary ETL.

The HBL (= a-ETL) may be free of the compound of formula (I).

### Electron transport layer (ETL)

The OLED according to the present invention comprises one or more electron transport layer(s) (ETL). In accordance with the invention, at least one of the electron transport layers is the inventive first electron transport layer comprising the compound of formula (I) as defined herein.

By suitably adjusting energy levels of particular layers of the ETL, the injection and transport of the electrons may be controlled, and the holes may be efficiently blocked. Thus, the OLED may have long lifetime.

The electron transport layer may comprise, besides the compound of formula (I) further ETM materials known in the art. Likewise, the electron transport layer may comprise as the only electron transport matrix material the compound of formula (I). In case that the inventive organic electronic device comprises more than one electron transport layers, the compound of formula (I) may be comprised in only one of the electron transport layers, in more than one of the electron transport layers or in all of the electron transport layers. Suitable further compounds for the ETM are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

### Electron injection layer (EIL)

The electron injection layer may be as described above. An EIL, which may facilitate injection of electrons from the cathode into the electron transport layer stack, may be formed on the electron transport layer stack, preferably directly on the electron transport layer stack, preferably directly on the second electron transport layer, preferably in direct contact with the second electron transport layer. Examples of materials for forming the EIL or being comprised in the EIL include lithium 8-hydroxyquinolinolate (LiQ), LiF, NaCl, CsF, Li₂O, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may comprise an organic matrix material doped with an n-type dopant. The matrix material may be selected from materials conventionally used as matrix materials for electron transport layers.

The EIL may consist of a number of individual EIL sublayers. In case the EIL consists of a number of individual EIL sublayers, the number of sublayers is preferably 2. The individual EIL sublayers may comprise different materials for forming the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode electrode

The cathode electrode is formed on the EIL if present, preferably directly on the EIL, preferably in direct contact with the EIL. In the sense of this invention the cathode and the EIL can be regarded as one functional part enabling the injection of electrons into the electron transport layer stack. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like, for example an alloy of Ag and Mg, such as Ag:Mg 90:10 wt/wt. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy. The transparent or semitransparent cathode may facilitate light emission through the cathode.

### Charge generation layer (CGL)

The charge generation layer (CGL) may comprise a p-type charge generation layer (p-CGL) and an n-type charge generation layer (n-CGL). An interlayer may be arranged between the p-CGL and the -n-CGL.

Typically, the charge generation layer is a p-n junction joining an n-type charge generation layer (electron generating layer) and a hole generating layer. The n-side of the p-n junction generates electrons and injects them into the layer that is adjacent in the direction to the anode. Analogously, the p-side of the p-n junction generates holes and injects them into the layer that is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluoro-7,7,8,8-tetracyanoquinodimethane (F₄-TCNQ), derivatives of tetracyanoquinodimethane, radialene derivatives, iodine, FeCl₃, FeF₃, and SbCl₅. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

According to one aspect of the present invention, the first electron transport layer is arranged between the first and second emission layer.

The CGL may be in direct contact with the first electron transport layer.

The n-CGL may be in direct contact with the first electron transport layer.

According to one aspect of the present invention, the first electron transport layer comprising the compound of formula (I) is arranged between a first and a second emission layer and a further first electron transport layer comprising the compound of formula (I) is arranged between the second emission layer and the cathode.

According to one aspect of the present invention, there is provided an organic light-emitting diode (OLED) comprising: a substrate; an anode electrode formed on the substrate; a hole injection layer, a hole transport layer, an emission layer, a first electron transport layer comprising the compound of formula (I) and a cathode electrode.

According to another aspect of the present invention, there is provided an OLED comprising: a substrate; an anode electrode formed on the substrate; a hole injection layer, a hole transport layer, an electron blocking layer, an emission layer, an auxiliary electron transport layer, a first electron transport layer comprising the compound of formula (I) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, a first electron transport layer comprising the compound of formula (I), an electron injection layer, and a cathode electrode.

According to various embodiments of the present invention, there may be provided OLEDs layers arranged between the above-mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to the first electron transport layer comprising the compound of formula (I), the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

In one embodiment, the organic light emitting diode according to the invention further comprises a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb): 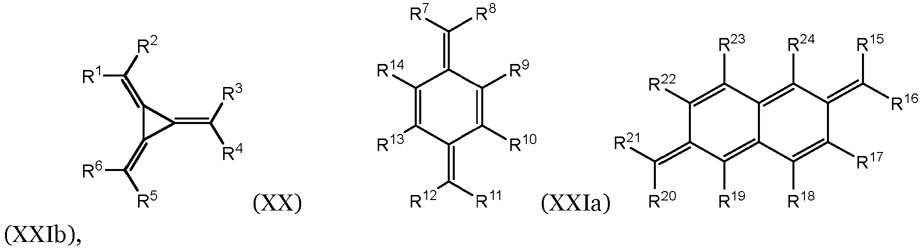 wherein (as an exception different to the description above) R¹, R², R³, R⁴, R⁵, R⁶, R⁷, R⁸, R¹¹, R¹², R¹⁵, R¹⁶, R²⁰, R²¹ are independently selected from above mentioned electron withdrawing groups and R⁹, R¹⁰, R¹³, R¹⁴, R¹⁷, R¹⁸, R¹⁹, R²², R²³ and R²⁴ are independently selected from H, halogen and above mentioned electron withdrawing groups.

### Process for preparing the organic electronic device

According to a further aspect, the invention is related to a process for preparing the organic light emitting diode according to the present invention, wherein the process comprises a step of depositing the compound of formula (I) according to the present invention on a solid support.

The method for depositing may comprise:
- deposition via vacuum thermal evaporation;
- deposition via solution processing, preferably the processing is selected from spin-coating, printing, casting; and/or
- slot-die coating.

### Display device

According to a further aspect, the invention is related to a display device comprising the organic light emitting diode according to the invention, preferably comprises at least two organic light emitting diodes according to the invention.

The display device may be a television, a tablet, or a mobile phone.

### GENERAL DEFINITIONS

If not explicitly mentioned else, each moiety of the compounds described herein, especially the compounds of formulas (I) may be substituted with one or more D (deuterium) and/or C₁ to C₄ alkyl.

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, C₃-alkyl may be selected from n-propyl and iso-propyl. Likewise, C₄-alkyl encompasses n-butyl, sec-butyl and t-butyl. Likewise, C₆-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in Cₙ relates to the total number of carbon atoms in the respective alkyl, arylene, heteroarylene or aryl group.

The term "aryl" or "arylene" as used herein shall encompass phenyl (C₆-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzene groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrenyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro [fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group -CR¹ = CR²R³ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in Cₙ-heteroaryl merely refers to the number of carbon atoms excluding the number of heteroatoms. In this context, it is clear that a C₃ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In accordance with the present disclosure, as an illustrative example using an arbitrary group A in a formula showing the following binding situation, 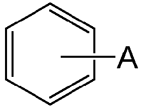
the group A may be bound to any suitable binding position. In a situation where it is shown that the bond of A crosses more than one ring 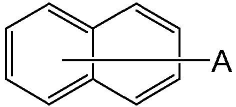
the group A may be bound to any suitable binding position of each ring crossed with the bond.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light through a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are used simultaneously and have the same meaning. The term "organic electroluminescent device" as used herein may comprise both organic light emitting diodes as well as organic light-emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the first electron transport layer comprising the compound of Formula (I) is essentially non-emissive or non-emitting.

The operating voltage, also named U, is measured in Volt (V) at 10 milliampere per square centimeter (mA/cm²).

The candela per ampere efficiency, also named cd/A efficiency or C_{eff}, is measured in candela per ampere at 10 milliampere per square centimeter (mA/cm²).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

Hereinafter, the embodiments are illustrated in more detail with reference to examples. However, the present disclosure is not limited to the following examples. Reference will now be made in detail to the exemplary aspects.

### DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures, which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic semiconducting device, according to an exemplary embodiment of the present invention;

FIG. 2 is a schematic sectional view of an organic light-emitting diode (OLED), according to an exemplary embodiment of the present invention;

FIG. 3 is a schematic sectional view of an OLED, according to an exemplary embodiment of the present invention.

FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

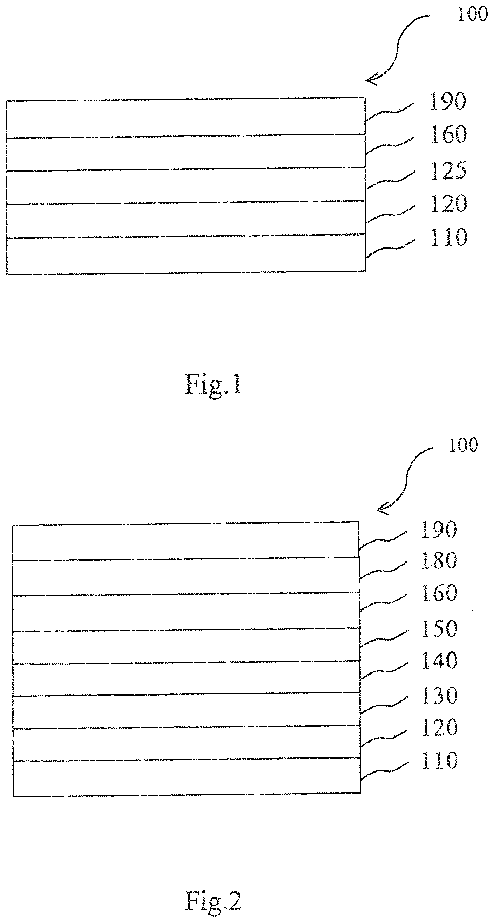

FIG. 1 is a schematic sectional view of an organic semiconducting device 100, according to an exemplary embodiment of the present invention. The organic semiconducting device 100 includes a substrate 110, an anode 120, a light emission layer (EML) 125, a first electron transport layer comprising or consisting of the compound of formula (I) according to the invention 160. The first electron transport layer comprising or consisting of the compound of formula (I) according to the invention 160 is formed on the EML 125. Onto the organic semiconductor layer 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an first electron transport layer (ETL) 160. The first electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

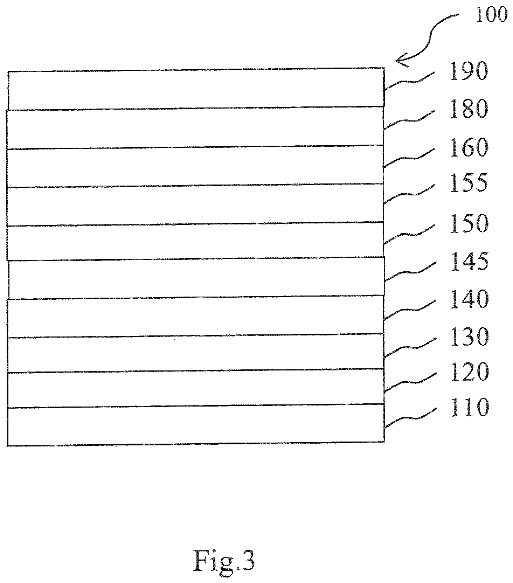

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, a first electron transport layer (ETL) 160, an electron injection layer (EIL) 180 and a cathode electrode 190.

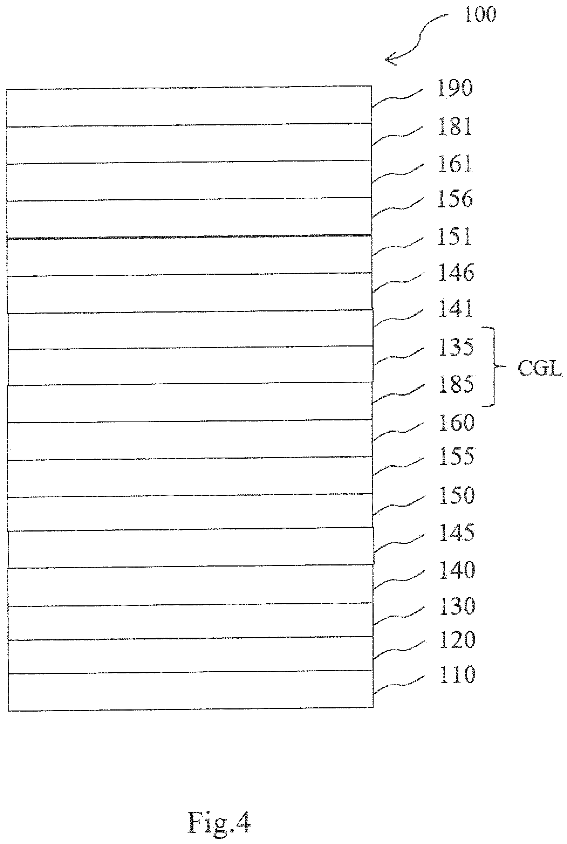

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL) and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

### EXPERIMENTAL PART

### Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 µL Mettler Toledo aluminum pan with lid, a <1 mm hole is pierced into the lid).

### Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

### Rate onset temperature

The rate onset temperature (T_{RO}) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

### Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc⁺/Fc redox couple, afforded finally the values reported above. All studied compounds as well as the reported comparative compounds showed well-defined reversible electrochemical behaviour.

### Dipole moment

The dipole moment |*̅µ̅*̅| of a molecule containing N atoms is given by: 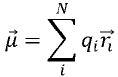 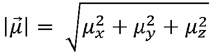 where *qᵢ* and *̅r̅ᵢ̅*̅ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

### Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

### Synthesis of compounds

### 6,6'-(4'-(tert-butyl)-[1,1':2',1"-terphenyl]-4,4"-diyl)bis(2,4-diphenyl-1,3,5-triazine) (Compound E-1)

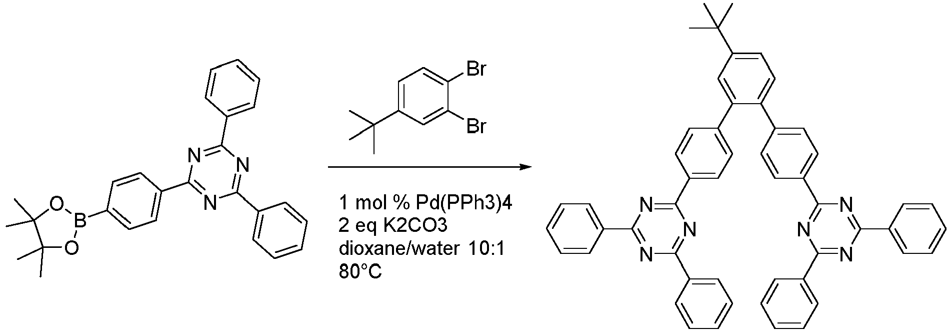

3 Neck round-bottom flask was flushed with nitrogen and charged with 1,2-dibromo-4-(tert-butyl)benzene (CAS 6683-75-6) 1 eq (9,5 g), 2,4-diphenyl-6-(3-(4,4,5,5-tetramethyl-1,3,2-dioxaborolan-2-yl)phenyl)-1,3,5-triazine (CAS 1269508-31-7) 2,1 eq (29,7 g), tetrakis(triphenylphosphine)palladium(o) (CAS 14221-01-3) 0,01 eq (0,4 g), potassium carbonate (CAS 584-08-7) 2 eq (9,0 g). Deaerated mixture of 330 ml dioxane and 33 ml water was added. Reaction was running at 80°C under a nitrogen atmosphere overnight. After cooling down to room temperature gray suspension was formed. Raw solid material was filtered, washed with water. Product was dissolved in 850 ml DCM, dried over MgSO₄ and filtered through a silica pad. The solvent was evaporated at low pressure. Next the product was recrystallized from toluene. Final purification was done by sublimation. White powder. Yield: 4,0 g (17 %). (ESI- MS: 749).

### 6,6'-(4'-(tert-butyl)-[1,1':2',1"-terphenyl]-3,3"-diyl)bis(2,4-diphenyl-1,3,5-triazine) (Compound E-2)

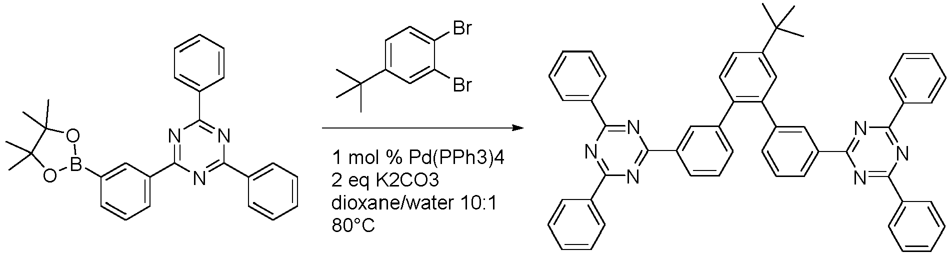

3 Neck round-bottom flask was flushed with nitrogen and charged with 1,2-dibromo-4-(tert-butyl)benzene (CAS 6683-75-6) 1 eq (9,5 g), 2,4-diphenyl-6-(3-(4,4,5,5-tetramethyl-1,3,2-dioxaborolan-2-yl)phenyl)-1,3,5-triazine (CAS 1269508-31-7) 2,1 eq (29,7 g), tetrakis(triphenylphosphine)palladium(o) (CAS 14221-01-3) 0,01 eq (0,4 g), potassium carbonate (CAS 584-08-7) 2 eq (9,0 g). Deaerated mixture of 330 ml dioxane and 33 ml water was added. Reaction was running at 80°C under a nitrogen atmosphere overnight. After cooling down to room temperature gray suspension was formed. Raw solid material was filtered, washed with water and methanol. Product was dissolved in 800 ml DCM and filtered through a silica pad, solvent was evaporated at low pressure. Next the product was recrystallized from toluene. Final purification was done by sublimation. White powder. Yield: 14,2 g (60 %). (ESI-MS: 749).

### 6,6'-(4'-(trimethylsilyl)-[1,1':2',1"-terphenyl]-4,4"-diyl)bis(2,4-diphenyl-1,3,5-triazine) (Compound E-6)

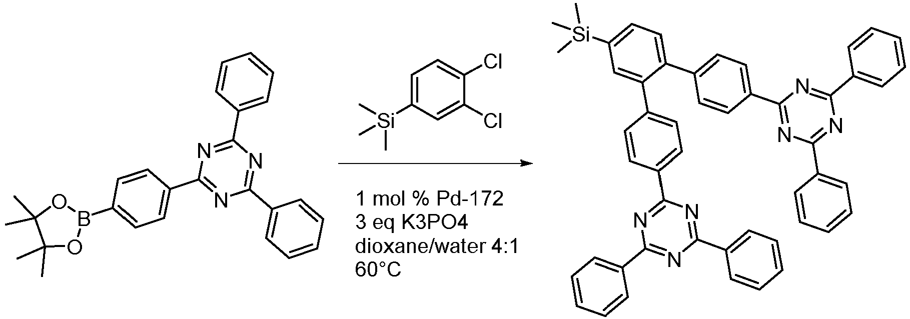

3 Neck round-bottom flask was flushed with nitrogen and charged with 1-(trimethylsilyl)-3,4-dichlorobenzene (CAS 149021-03-4) 1 eq (5,0 g), 2,4-diphenyl-6-(4-(4,4,5,5-tetramethyl-1,3,2-dioxaborolan-2-yl)phenyl)-1,3,5-triazine (CAS 1219956-23-6) 2 eq (19,9 g), chloro(crotyl)(2-dicyclohexylphosphino-2',6'-dimethoxybiphenyl)palladium(II) (CAS 1798781-99-3 ) 0,01 eq (0,3 g), potassium phosphate (CAS 7778-53-2) 3 eq (29,0 g). Deaerated mixture of 270 ml dioxane and 70 ml water was added. Reaction was running at 60°C under a nitrogen atmosphere overnight. After cooling down to room temperature thick gray suspension was formed. Raw solid material was filtered, washed with water. Product was dissolved in 700 ml chloroform, dried over MgSO₄ and filtered through a silica pad. Next the product was recrystallized from toluene/chlorobenzene mixture (Vol. 1:1). Final purification was done by sublimation. White powder. Yield: 11,7 g (64 %). (ESI- MS: 765).

### 6,6'-(4'-(trifluoromethyl)-[1,1':2',1"-terphenyl]-3,3"-diyl)bis(2,4-diphenyl-1,3,5-triazine) (Compound E-14)

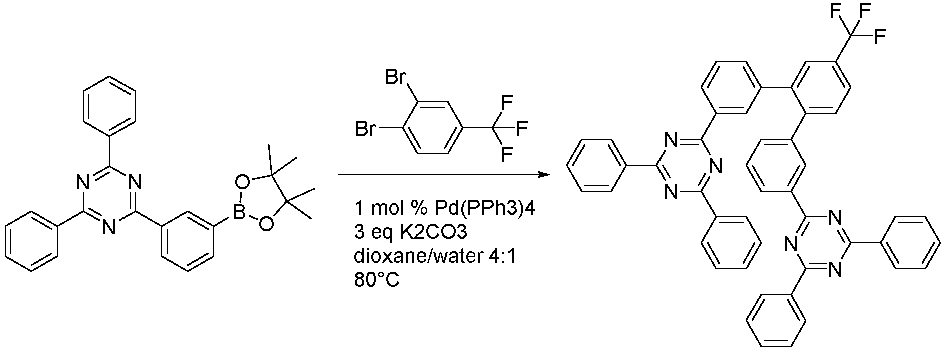

3 Neck round-bottom flask was flushed with nitrogen and charged with 1,2-dibromo-4-(trifluoromethyl)benzene (CAS 7657-08-1) 1 eq (3,0 g), 2,4-diphenyl-6-(3-(4,4,5,5-tetramethyl-1,3,2-dioxaborolan-2-yl)phenyl)-1,3,5-triazine (CAS 1269508-31-7) 2 eq (8,9 g), tetrakis(triphenylphosphine)palladium(o) (CAS 14221-01-3) 0,01 eq (0,2 g), potassium carbonate (CAS 584-08-7) 3 eq (4,2 g). Deaerated mixture of 60 ml dioxane, 15 ml water was added. Reaction was running at 80°C under a nitrogen atmosphere overnight. After cooling down to room temperature white suspension was formed. Raw solid material was filtered, washed with dioxane and water. Product was dissolved at elevated temperature in 190 ml chlorobenzene and filtered hot through a silica pad. After cooling the extract down white precipitate was formed, filtered and washed with hexane. Final purification was done by sublimation. White powder. Yield: 5,4 g (72 %). (ESI- MS: 761)

### 6,6'-(4',5'-difluoro-[1,1':2',1"-terphenyl]-3,3"-diyl)bis(2,4-diphenyl-1,3,5-triazine) (Compound E-15)

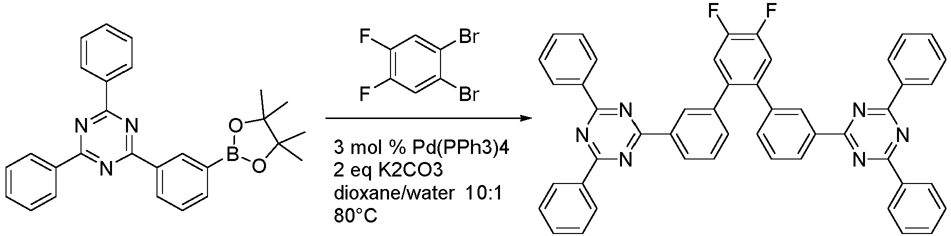

3 Neck round-bottom flask was flushed with nitrogen and charged with 1,2-dibromo-4,5-difluorobenzene (CAS 64695-78-9) 1 eq (9,4 g), 2,4-diphenyl-6-(3-(4,4,5,5-tetramethyl-1,3,2-dioxaborolan-2-yl)phenyl)-1,3,5-triazine (CAS 269410-07-3) 2 eq (30,0 g), tetrakis(triphenylphosphine)palladium(o) (CAS 14221-01-3) 0,03 eq (1,2g), potassium carbonate (CAS 584-08-7) 2 eq (6,7 g). Deaerated mixture of 350 ml dioxane and 35 ml water was added. Reaction was running at 80°C under a nitrogen atmosphere overnight. After cooling down to room temperature yellow suspension was formed. Raw solid material was filtered, washed with methanol and water. Product was dissolved at elevated temperature in 700 ml mixture of chlorobenzene/DMF (vol. 6:1) and filtered hot through a silica pad. Then the solvent was evaporated, the product was stirred in 300 ml THF at room temperature and filtered. Final purification was done by sublimation. White powder. Yield: 19,2 g (78 %). (ESI- MS: 729)

### 6,6'-(4'-(tert-butyl)-[1,1':2',1"-terphenyl]-3,4"-diyl)bis(2,4-diphenyl-1,3,5-triazine) (compound E-16)

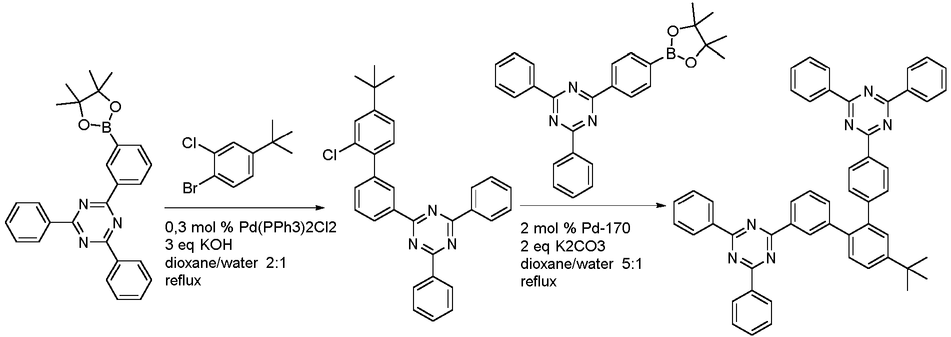

### 1^{st} Step. 2-(4'-(tert-butyl)-2'-chloro-[1,1'-biphenyl]-3-yl)-4,6-diphenyl-1,3,5-triazine

3 Neck round-bottom flask was flushed with nitrogen and charged with 1-bromo-4-(tert-butyl)-2-chlorobenzene (CAS 1251032-65-1) 1 eq (12,4 g), 2,4-diphenyl-6-(3-(4,4,5,5-tetramethyl-1,3,2-dioxaborolan-2-yl)phenyl)-1,3,5-triazine (CAS 1269508-31-7) 1,3 eq (28,3 g), bis(triphenylphosphine)-palladium-II-chloride (CAS 13965-03-2) 0,003 eq (0,1 g), potassium hydroxide (solid) (CAS 1310-58-3) 3 eq (8,4 g). Deaerated mixture of 300 ml dioxane and 150 ml water was added. Reaction was running at reflux under a nitrogen atmosphere overnight. After cooling down to room temperature gray suspension was formed. Raw solid material was filtered, washed with dioxane. Product was dissolved in 410 ml chloroform and filtered through a silica pad. Then the solvent was partially evaporated and 250 ml of 2-methoxy-2-methylpropane was added. White precipitate was formed and filtered. Yield: 15,7 g (68 %).

### 2^{nd} Step. 6,6'-(4'-(tert-butyl)-[1,1':2',1"-terphenyl]-3,4"-diyl)bis(2,4-diphenyl-1,3,5-triazine)

3 Neck round-bottom flask was flushed with nitrogen and charged with 2-(4'-(tert-butyl)-2'-chloro-[1,1'-biphenyl]-3-yl)-4,6-diphenyl-1,3,5-triazine (no CAS) 1 eq (3,1 g), 2,4-diphenyl-6-(4-(4,4,5,5-tetramethyl-1,3,2-dioxaborolan-2-yl)phenyl)-1,3,5-triazine (CAS 1219956-23-6) 1,1 eq (3,1 g), chloro(crotyl)(2-dicyclohexylphosphino-2',4',6'-triisopropybiphenyl)palladium(II) (CAS 1798782-02-1) 0,02 eq (0,1 g), potassium carbonate (CAS 584-08-7) 2 eq (1,8 g). Deaerated mixture of 35 ml dioxane and 7 ml water was added. Reaction was running at reflux under a nitrogen atmosphere overnight. After cooling down to room temperature thick gray suspension was formed. Raw solid material was filtered, washed with dioxane, water and methanol. Product was dissolved at elevated temperature in 100 ml chlorobenzene and filtered hot through a silica pad. After cooling the extract down white precipitate was formed, filtered and washed with hexane. Final purification was done by sublimation. White powder. Yield: 3,6 g (75%). (ESI- MS: 749)

**Table 1: Properties of the synthesized compounds (DFT data Turbomole, SDC standard procedure (B3LYP-Gaussian / 6-31G*, gas phase))**

| | **HOMO [eV]** | **LUMO [eV]** | **E gap [eV]** | **Dipole [Debye]** |
|---|---|---|---|---|
| C-1 (comparative) | -6,03 | -1,92 | 4,11 | 0,23 |
| E-1 | -5,92 | -1,90 | 4,02 | 0,90 |
| E-2 | -5,94 | -1,79 | 4,15 | 0,52 |
| E-6 | -5,98 | -1,92 | 4,06 | 0,59 |
| E-14 | -6,29 | -1,91 | 4,38 | 2,93 |
| E-15 | -6,12 | -1,91 | 4,21 | 2,81 |
| E-16 | -5,92 | -1,84 | 4,08 | 0,68 |

**Table 2: Thermal and CV data of inventive compounds**

| | **Mp [°C]** | **Tg [°C]** | **Rate onset [°C]** |
|---|---|---|---|
| | | | (test chamber; bottom sensor) |
| C-1 (comparative) | 348 | 138 | 297 |
| E-6 | 320 | 151 | 268 |
| E-1 | 313 | 155 | 267 |
| E-2 | 281 | 134 | 243 |
| E-16 | 341 | 143 | 270 |
| E-15 | 309 | 123 | 247 |
| E-14 | 318 | not observed | 249 |

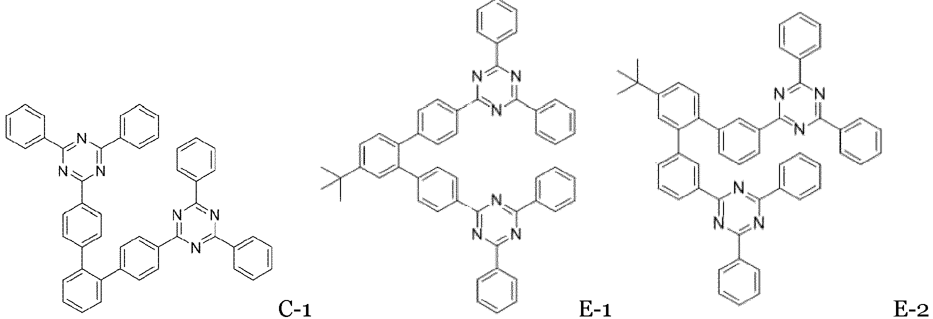

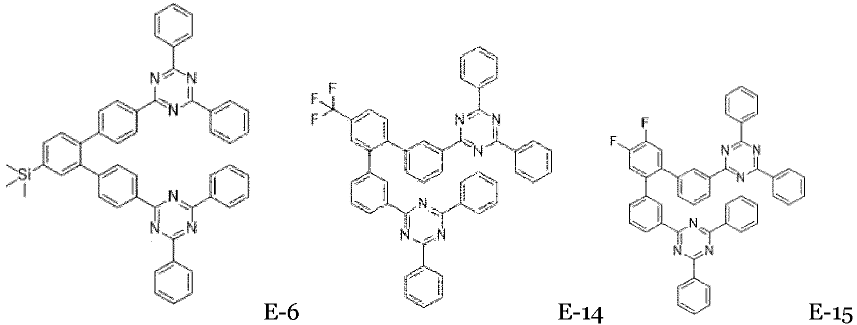

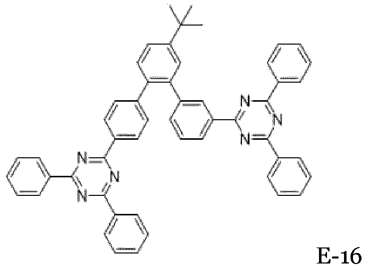

### Device experiments

### Procedure to manufacture the top emission fluorescent blue OLED

For the top emission OLED devices an ITO / Ag / ITO substrate with dimensions of 100 mm x 100 mm x 0.7 mm was ultrasonically cleaned with DI water rinse 20 minutes, then with Isopropanol for 20 minutes in beaker, and dried for 15 minutes in a spin rinse dryer. Subsequently, the substrate was baked out in oven at 200°C for ~2h. Before organic material deposition it gets plasma treatment in vacuum chamber (typically N₂:O₂ plasma).

The device was made by depositing a hole injection layer by co-deposition of HT-1 and D-1 onto substrate provided with ITO / Ag / ITO anode, followed by a undoped hole transport layer of HT-1. Subsequently, an electron blocking layer of HT-2 was deposited onto the HTL. Subsequently, a blue fluorescent emitting layer of emitter host Host-1 doped with Emitter-1 was deposited. On the emission layer, a layer made of ET-1 was deposited as a hole blocking layer. An electron transport layer made by co-deposition of the comparative compound ET-2:LiQ or the inventive compounds:LiQ onto the HBL. Subsequently, an electron injection layer made of Yb was deposited onto the ETL followed by a cathode consisting of a silver-magnesium alloy in a volume ratio 90:10. Finally, a capping layer made of HT-3 was deposited on top of the cathode. All depositions were made by vacuum thermal evaporation.

OLED device structure: ITO, 10nm /Ag, 120nm /ITO, 8nm / HT-1:D-1 (98:2 wt%), 10nm / HT-1, 129nm / HT-2, 5nm / Host-1 :Emitter-1 (99:1 vol%), 20nm / ET-1, 5nm / FORMULA (I) or comparative ET-2:LiQ (50:50 vol%), 31nm / Yb, 1.3nm / Ag:Mg (90:10 vol%), 13nm / HT-3, 75nm

**Table 3: List of compounds used**

| | IUPAC name | Reference |
|---|---|---|
| HT-1 | N-(9,9-diphenyl-9H-fluoren-2-yl)-N,9-diphenyl-9H-carbazol-2-amine | - |
| | CAS 1607480-22-7 | |
| D-1 | 4,4',4"-((1E,1'E,1"E)-cyclopropane-1,2,3-triylidenetris(cyanomethanylylidene))tris(2,3,5,6-tetrafluorobenzonitrile) | US2008265216 |
| | CAS 1224447-88-4 | |
| HT-2 | N,N-di([1,1'-biphenyl]-4-yl)-3'-(9H-carbazol-9-yl)-[1,1'-biphenyl]-4-amine | - |
| | CAS 1464822-27-2 | |
| Host-1 | CAS 2457172-82-4 | - |
| Emitter-1 | CAS 2482607-57-6 | - |
| ET-1 | 4-([1,1'-biphenyl]-4-yl)-6-(3'-(9,9-dimethyl-9H-fluoren-4-yl)-[1,1'-biphenyl]-4-yl)-2-phenylpyrimidine | WO2016105141 |
| | CAS 1955546-40-3 | |
| HT-3 | N-([1,1'-biphenyl]-4-yl)-9,9-dimethyl-N-(4-(9-phenyl-9H-carbazol-3-yl)phenyl)-9H-fluoren-2-amine | US2016322581 |
| | CAS 1242056-42-3 | |
| LiQ | 8-Hydroxyquinolinolato-lithium | - |
| | CAS 850918-68-2 | |
| ET-2 | 4,4"-bis(4,6-diphenyl-1,3,5-triazin-2-yl)-1,1':2',1"-terphenyl | EP3739645 |
| | CAS 2230476-56-7 | |

**Table 4: Device performance**

| **1^{st} ETL** | **CIEy** | **Voltage, 10mA/cm² [V]** | | **Rel. Ceff/CIEy, 10 mA/cm² [%]** |
|---|---|---|---|---|
| ET-2 :LiQ (comparative) | 0.050 | 3.37 | | 100.0 |
| E-1:LiQ | 0.050 | 3.40 | | **102.4** |
| E-2:LiQ | 0.050 | 3.46 | | **102.9** |
| E-6:LiQ | 0.049 | 3.39 | | **103.0** |
| E-15:LiQ | 0.050 | 3.86 | | **102.6** |

### Technical Effect of the invention

The OLED devices comprising the inventive compounds in the first ETL thereof show improved efficiency.

The features disclosed in the foregoing description and in the dependent claims may, both separately and in any combination thereof, be material for realizing the aspects of the disclosure made in the independent claims, in diverse forms thereof.

## Claims

1. Compound of formula (I) 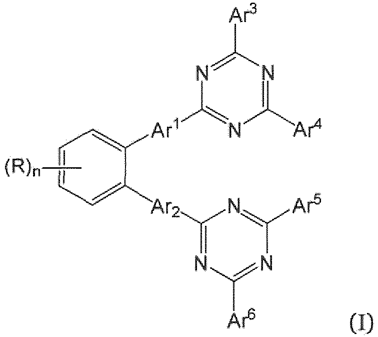 wherein
n is an integer from 1 to 4;
R is independently selected from the group consisting of C₁ to C₁₂ alkyl, partially fluorinated C₁ to C₁₂ alkyl, perfluorinated C₁ to C₁₂ alkyl, F, and SiR'₃, wherein R' is independently selected from C₁ to C₁₂ alkyl;
Ar¹ and Ar² are independently selected C₆ to C₁₂ arylene; and
Ar³ to Ar⁶ are independently selected C₆ to C₁₂ aryl.

2. Compound according to claim 1, wherein the compound of formula (I) has the formula (Ia) 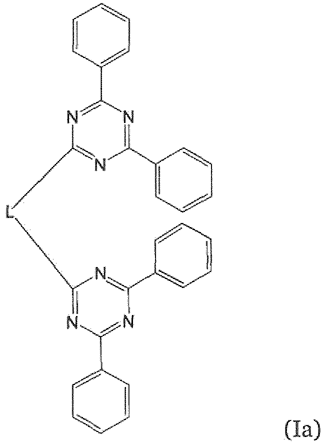
wherein L is independently selected from L-1 to L-5 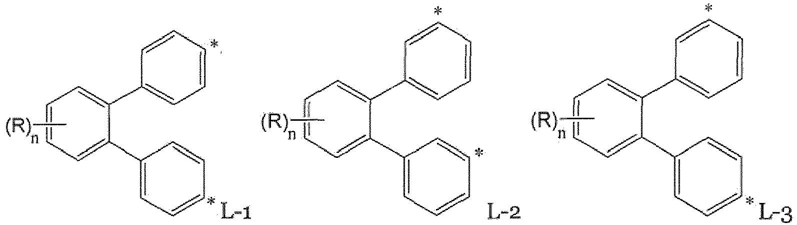 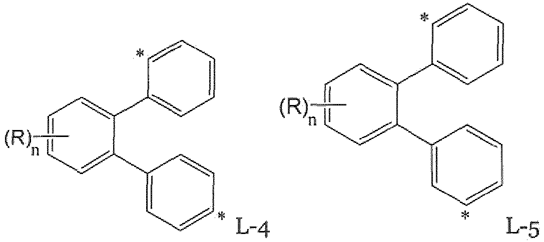
wherein the * represent the binding positions.

3. Compound according to claim 1 or 2, wherein n is 1 or 2.

4. Compound according to any of the preceding claims, wherein R is independently selected from the group consisting of C₁ to C₆ alkyl, partially deuterated C₂ to C₆ alkyl, partially fluorinated C₁ to C₆ alkyl, perfluorinated C₁ to C₆ alkyl, F, and SiR'₃, wherein R' is independently selected from C₁ to C₆ alkyl.

5. Compound according to any of the preceding claims, wherein R is independently selected from the group consisting of C₁ to C₄ alkyl, partially fluorinated C₁ to C₄ alkyl, perfluorinated C₁ to C₄ alkyl, F, and SiR'₃, wherein R' is independently selected from C₁ to C₄ alkyl.

6. Compound according to any of the preceding claims, wherein R is independently selected from the group consisting of R-1 to R-6 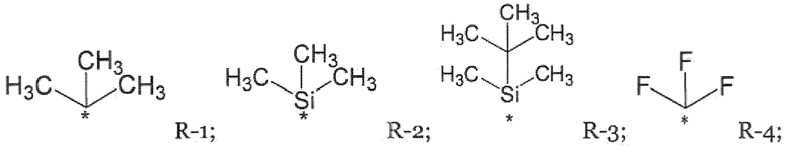
F*_{R-5}; * CH_{3_{R-6}}.
wherein the * represent the binding position.

7. Compound according to any of the preceding claims, wherein the compound is selected from E-1 to E-28

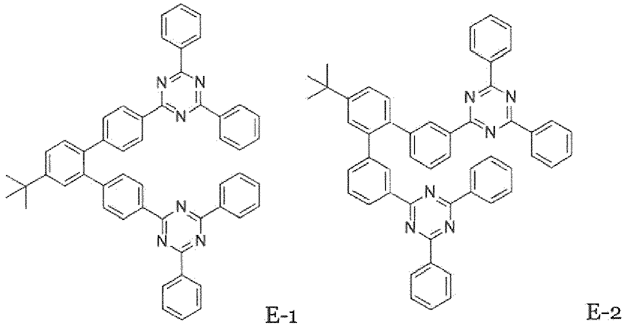

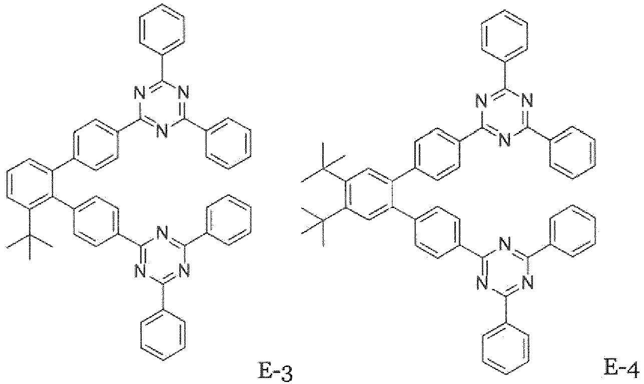

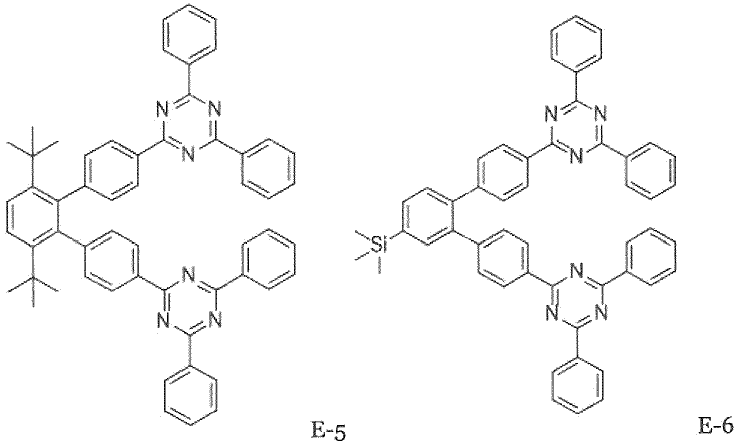

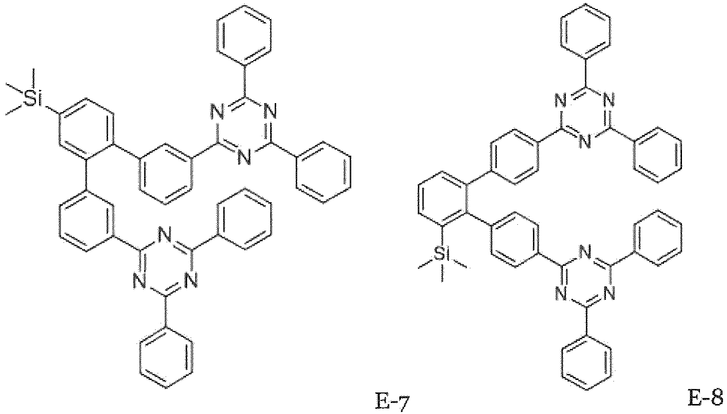

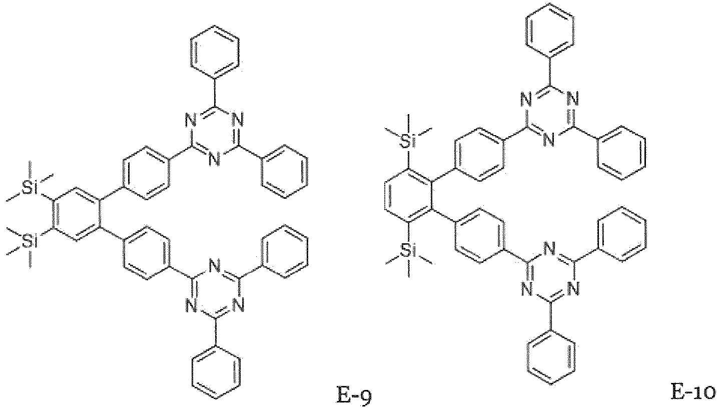

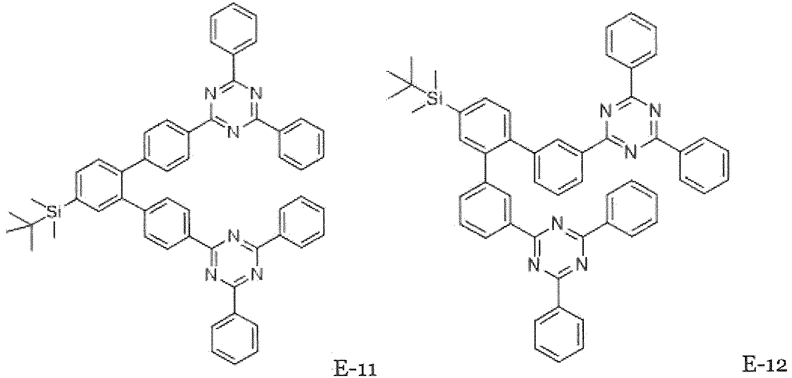

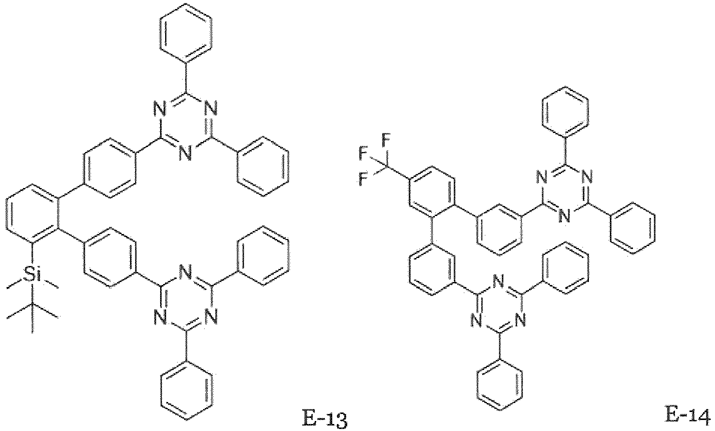

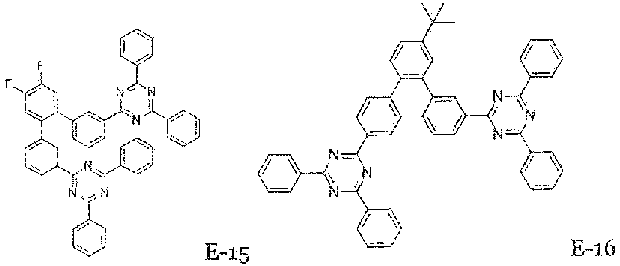

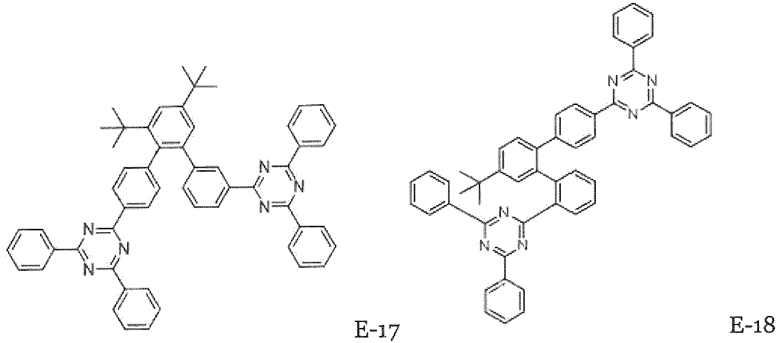

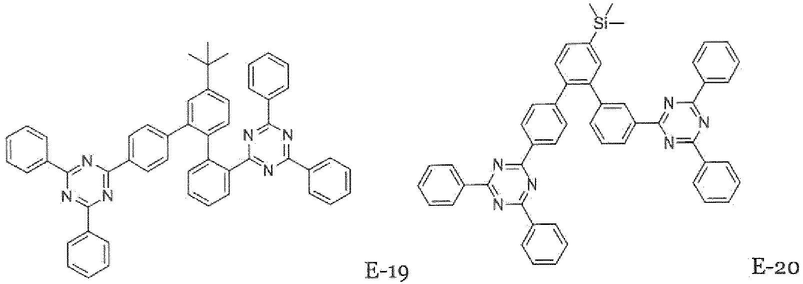

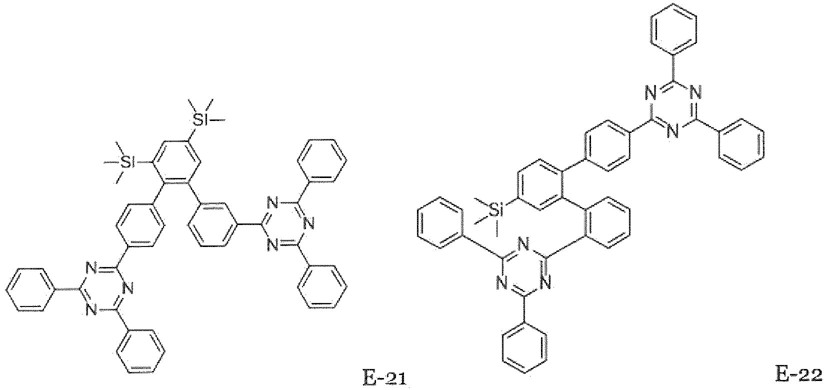

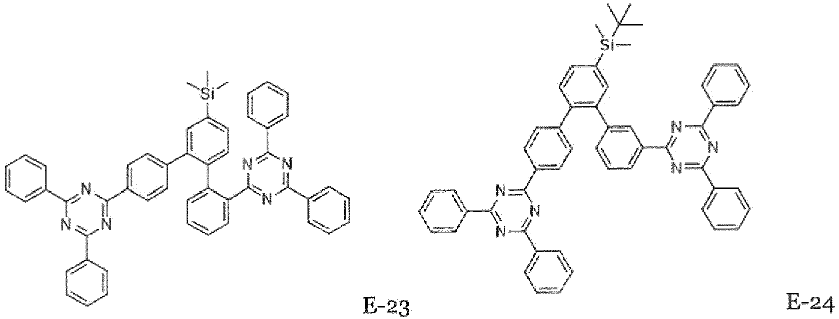

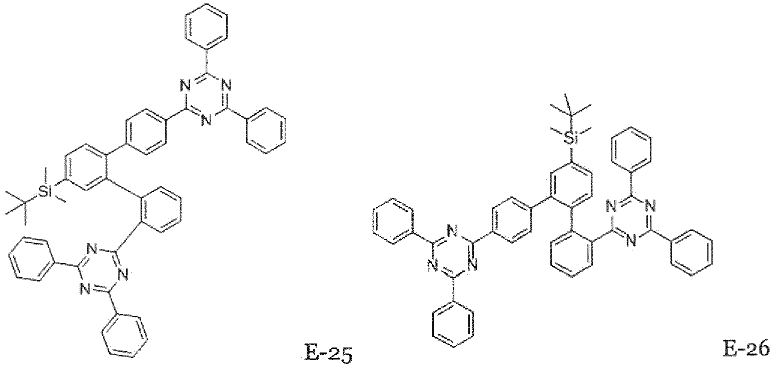

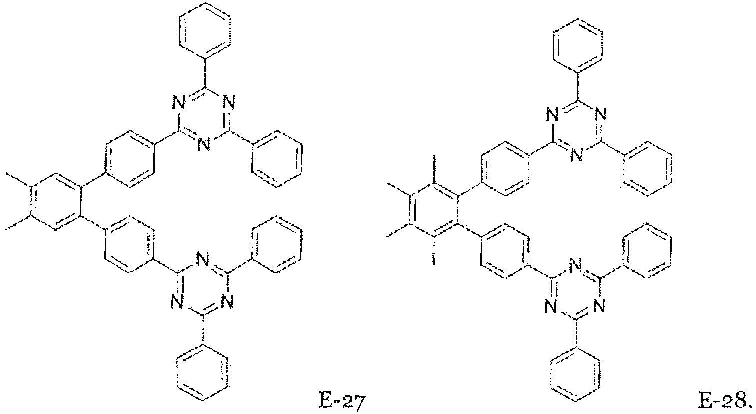

8. Organic light emitting diode comprising a substrate (110), an anode (120), a cathode (190), an emission layer (125), and a first electron transport layer (160),
wherein
- the first electron transport layer is arranged between the emission layer and the cathode; and
- the first electron transport layer comprises the compound of formula (I) according to any of the preceding claims.

9. Organic light emitting diode according to claim 8, wherein the first electron tranpsort layer further comprises an n-type dopant.

10. Organic light emitting diode according to claim 8 or 9, wherein the organic light emitting diode further comprises an auxiliary electron transport layer and the auxiliary electron transport layer is arranged between the emission layer and the first electron transport layer.

11. Organic light emitting diode according to claim 10, wherein the first electron transport layer is contacting sandwiched between the auxiliary electron transport layer and the electron injection layer.

12. Organic light emitting diode according to any of the claims 8 to 11, wherein the organic light emitting diode further comprises an electron injection layer and the electron injection layer is arranged between the first electron tranpsort layer and the cathode.

13. Organic light emitting diode according to any of the claims 8 to 12, wherein the organic light emitting diode is a single stack organic light emitting diode or a multi-stack organic light emitting diode.

14. Display device comprising the organic light emitting diode according to any of the claims 8 to 13.

15. Display device according to claim 14, wherein the display device is a television, a tablet, or a mobile phone.

## Patentansprüche

1. Verbindung der Formel (I) 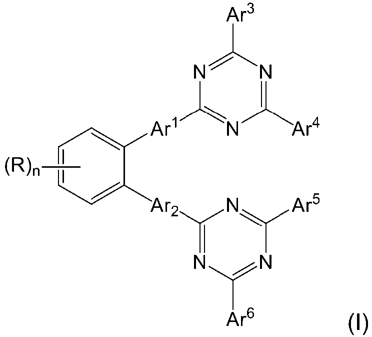 wobei
n eine ganze Zahl von 1 bis 4 ist;
R unabhängig ausgewählt ist aus der Gruppe bestehend aus C₁ - bis C₁₂ -Alkyl, teilweise fluoriertem C₁ - bis C₁₂ -Alkyl, perfluoriertem C₁ - bis C₁₂ -Alkyl, F und SiR'₃ , wobei R' unabhängig ausgewählt ist aus C₁ - bis C₁₂ -Alkyl;
Ar¹ und Ar² unabhängig ausgewähltes C₆ - bis C₁₂ -Arylen sind; und
Ar³ bis Ar⁶ unabhängig ausgewähltes C₆ - bis C₁₂ -Aryl sind.

2. Verbindung nach Anspruch 1, wobei die Verbindung der Formel (I) die Formel (la) aufweist 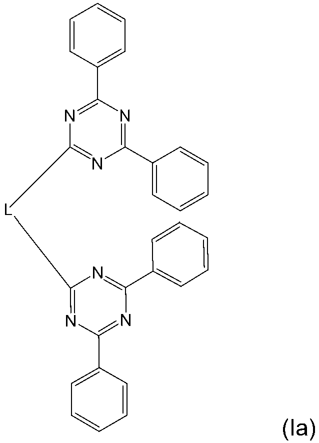
wobei L unabhängig ausgewählt ist aus L-1 bis L-5 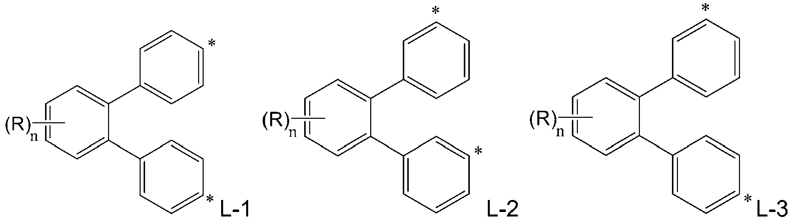 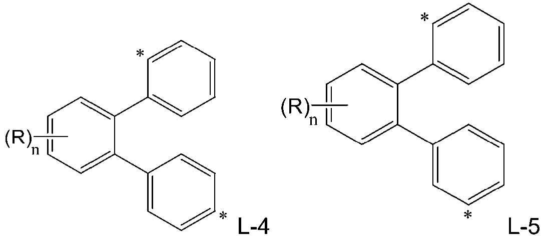
wobei die * die Bindungspositionen darstellen.

3. Verbindung nach Anspruch 1 oder 2, wobei n 1 oder 2 ist.

4. Verbindung nach einem der vorhergehenden Ansprüche, wobei R unabhängig ausgewählt ist aus der Gruppe bestehend aus C₁ - bis C₆ -Alkyl, teilweise deuteriertem C₁ - bis C₆ -Alkyl, teilweise fluoriertem C₁ - bis C₆ -Alkyl, perfluoriertem C₁ - bis C₆ -Alkyl, F und SiR'₃ , wobei R' unabhängig ausgewählt ist aus C₁ - bis C₆ -Alkyl.

5. Verbindung nach einem der vorhergehenden Ansprüche, wobei R unabhängig ausgewählt ist aus der Gruppe bestehend aus C₁ - bis C₄ -Alkyl, teilweise fluoriertem C₁ - bis C₄ -Alkyl, perfluoriertem C₁ - bis C₄ -Alkyl, F und SiR'₃ , wobei R' unabhängig ausgewählt ist aus C₁ - bis C₄ -Alkyl.

6. Verbindung nach einem der vorhergehenden Ansprüche, wobei R unabhängig ausgewählt ist aus der Gruppe bestehend aus R-1 bis R-6 , 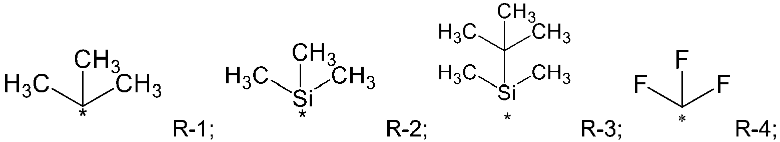
F*_{R-5}; * CH_{3_{R-6}}
wobei die * die Bindungsposition darstellen.

7. Verbindung nach einem der vorhergehenden Ansprüche, wobei die Verbindung ausgewählt ist aus E-1 bis E-28.

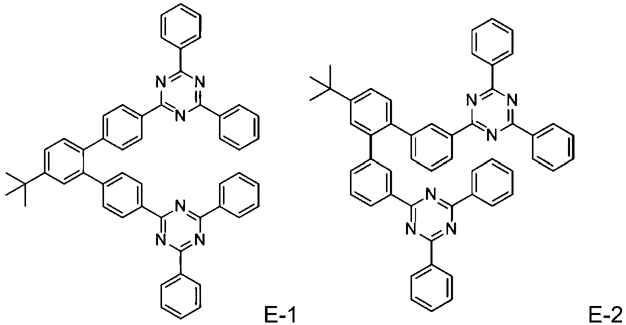

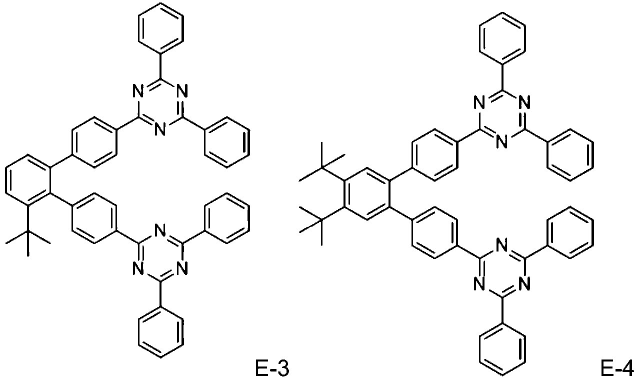

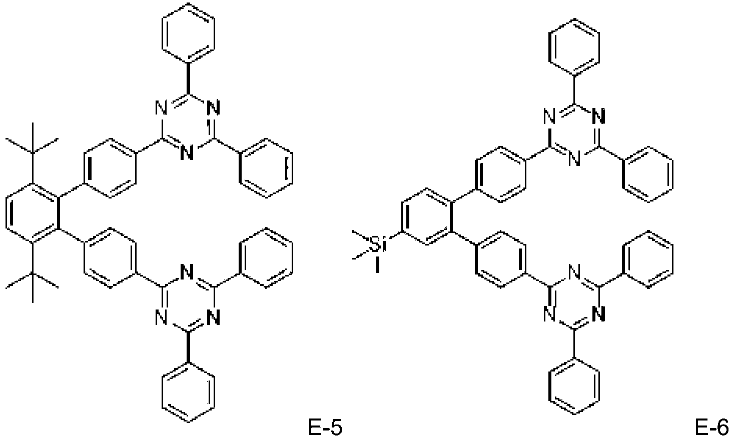

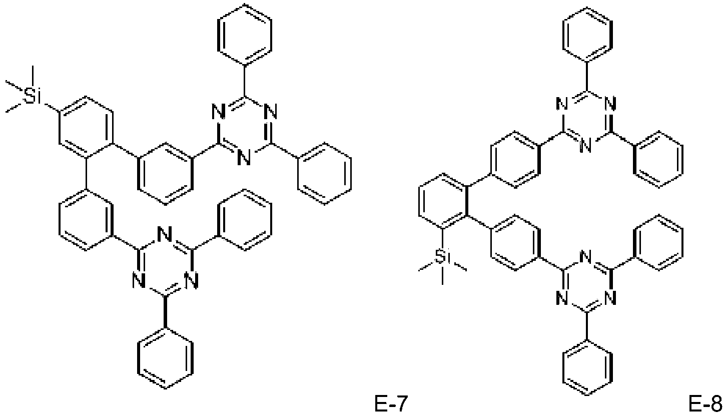

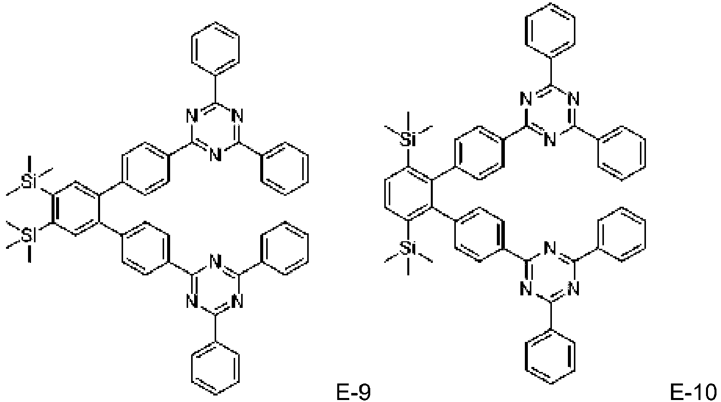

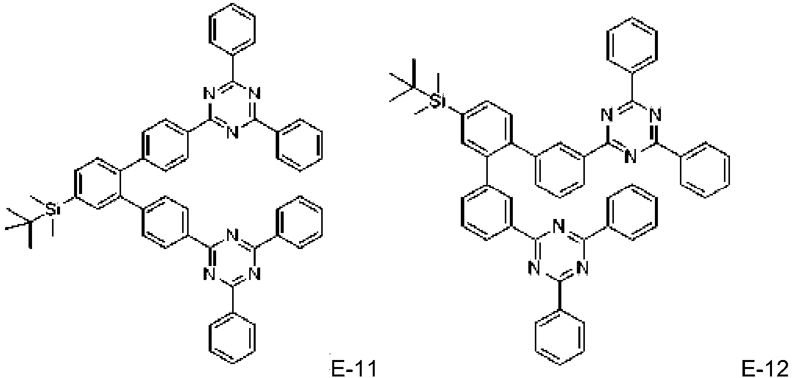

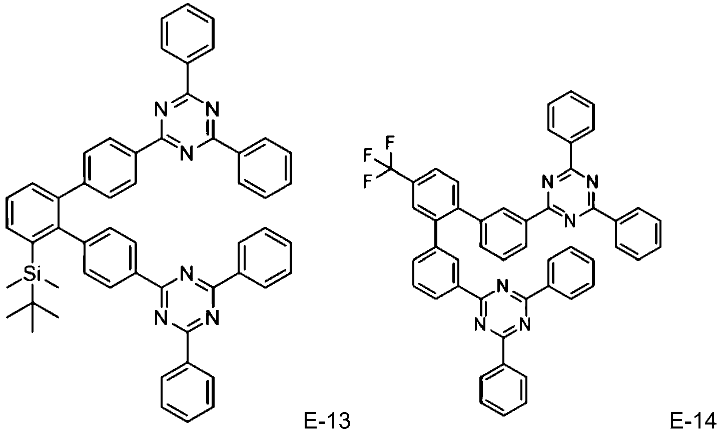

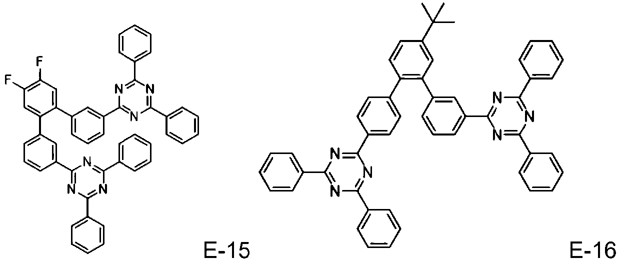

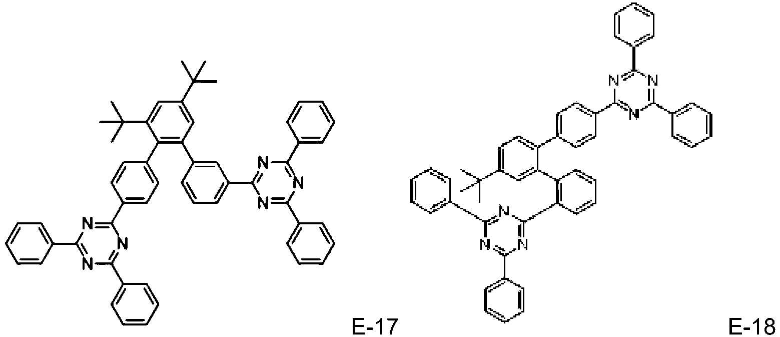

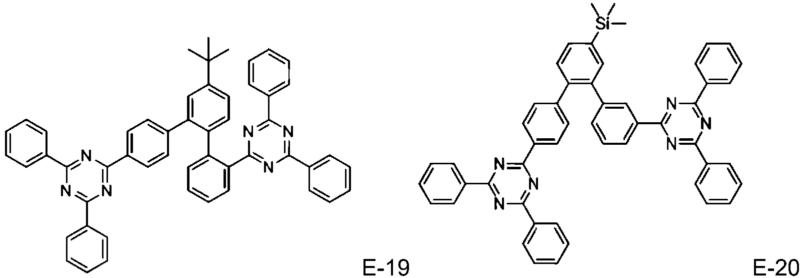

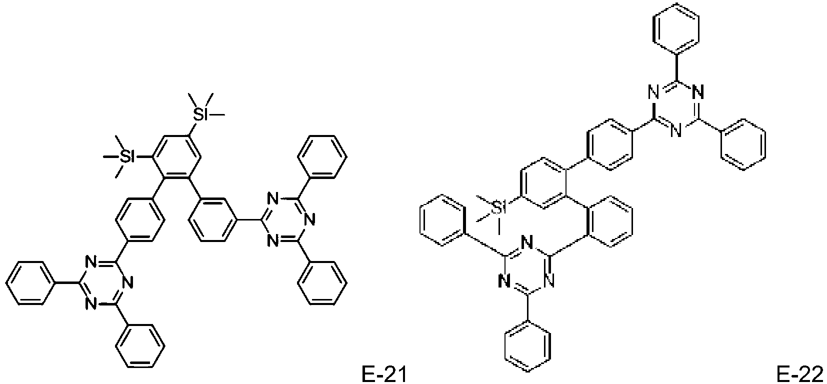

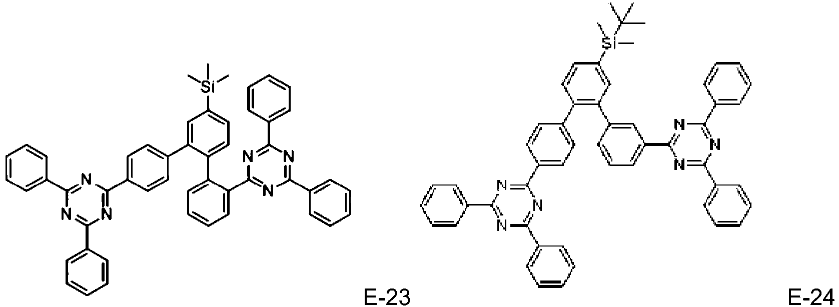

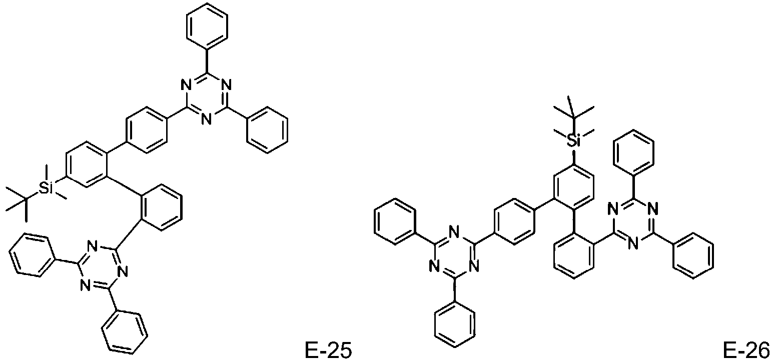

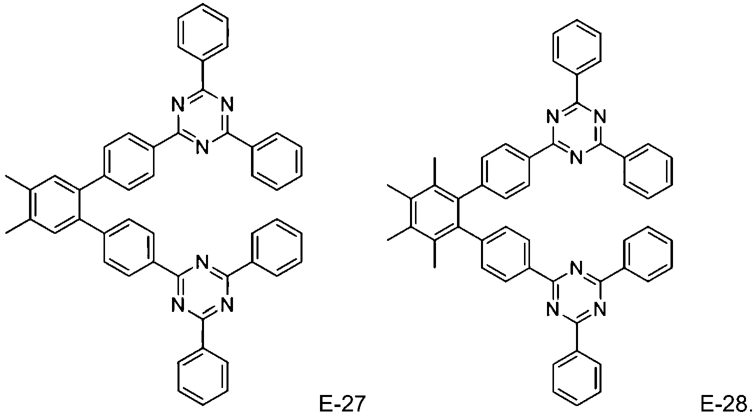

8. Organische Leuchtdiode, umfassend ein Substrat (110), eine Anode (120), eine Kathode (190), eine Emissionsschicht (125) und eine erste Elektronentransportschicht (160),
wobei
- die erste Elektronentransportschicht zwischen der Emissionsschicht und der Kathode angeordnet ist; und
- die erste Elektronentransportschicht die Verbindung der Formel (I) nach einem der vorhergehenden Ansprüche umfasst.

9. Organische Leuchtdiode nach Anspruch 8, wobei die erste Elektronentransportschicht ferner einen Dotierstoff vom n-Typ umfasst.

10. Organische Leuchtdiode nach Anspruch 8 oder 9, wobei die organische Leuchtdiode ferner eine Hilfselektronentransportschicht umfasst und die Hilfselektronentransportschicht zwischen der Emissionsschicht und der ersten Elektronentransportschicht angeordnet ist.

11. Organische Leuchtdiode nach Anspruch 10, wobei die erste Elektronentransportschicht sandwichartig zwischen der Hilfselektronentransportschicht und der Elektroneninjektionsschicht kontaktiert ist.

12. Organische Leuchtdiode nach einem der Ansprüche 8 bis 11, wobei die organische Leuchtdiode ferner eine Elektroneninjektionsschicht umfasst und die Elektroneninjektionsschicht zwischen der ersten Elektronentransportschicht und der Kathode angeordnet ist.

13. Organische Leuchtdiode nach einem der Ansprüche 8 bis 12, wobei die organische Leuchtdiode eine organische Einzelstapel-Leuchtdiode oder eine organische Mehrfachstapel-Leuchtdiode ist.

14. Anzeigevorrichtung, umfassend die organische Leuchtdiode nach einem der Ansprüche 8 bis 13.

15. Anzeigevorrichtung nach Anspruch 14, wobei die Anzeigevorrichtung ein Fernseher, ein Tablet oder ein Mobiltelefon ist.

## Revendications

1. Composé de formule (I) 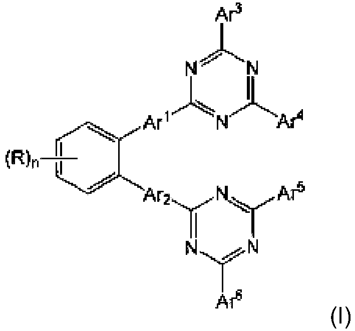 dans laquelle
n est un nombre entier compris entre 1 et 4 ;
R est choisi indépendamment dans le groupe constitué par les groupes alkyle en C₁ à C₁₂, les groupes alkyle en C₁ à C₁₂ partiellement fluorés, les groupes alkyle en C₁ à C₁₂ perfluorés, F et SiR'₃, dans laquelle R' est choisi indépendamment parmi les groupes arylène en C₄ à C₁₂ ; et
Ar¹ et Ar² sont choisis indépendamment parmi arylène en C₆ à C₁₂ ; et
Ar³ à Ar⁶ sont choisis indépendamment parmi aryle en C₆ à C₁₂.

2. Composé selon la revendication 1, dans lequel le composé de formule (1) répond à la formule (la) 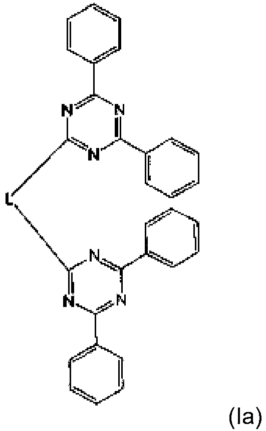
dans laquelle L est choisi indépendamment parmi L-1 à L-5 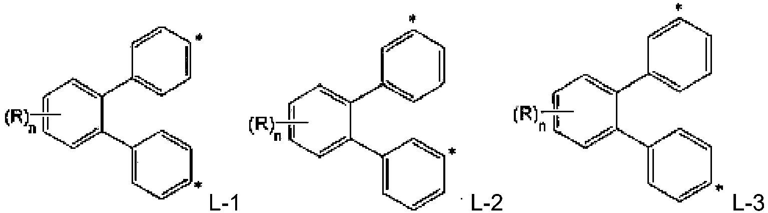 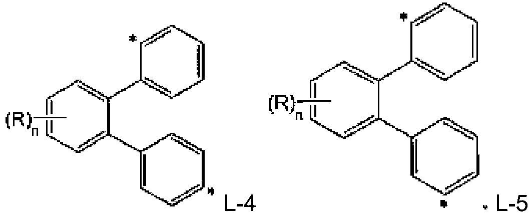
dans lesquels les * représentent les positions de liaison.

3. Composé selon la revendication 1 ou la revendication 2, dans lequel n est 1 ou 2.

4. Composé selon l'une quelconque des revendications précédentes, dans lequel R est choisi indépendamment dans le groupe constitué par les groupes alkyle en C₄ à C₆, les groupes alkyle en C₁ à C₆ partiellement deutérés, les groupes alkyle en C₁ à C₆ partiellement fluorés, les groupes alkyle en C₁ à C₆ perfluorés, F et SiR'₃, dans laquelle le groupe R' est choisi indépendamment parmi les groupes alkyle en C₁ à C₆.

5. Composé selon l'une quelconque des revendications précédentes, dans lequel R est choisi indépendamment dans le groupe constitué par les groupes alkyle en C₄ à C₄, les groupes alkyle en C₁ à C₄ partiellement fluorés, les groupes alkyle en C₁ à C₄ perfluorés, F et SiR'₃, dans laquelle le groupe R' est choisi indépendamment parmi les groupes alkyle en C₁ à C₄.

6. Composé selon l'une quelconque des revendications précédentes, dans lequel R est choisi indépendamment parmi le groupe constitué des groupes R-1 à R-6 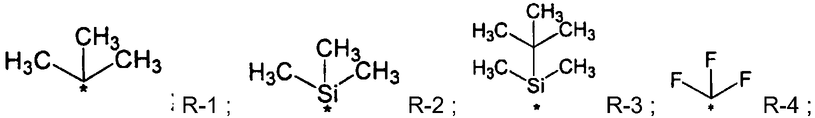
**F*** _{R-5} ; * CH_{3_{R-6}} ;
dans lesquels le symbole * représente la position de liaison.

7. Composé selon l'une quelconque des revendications précédentes, dans lequel le composé est choisi parmi les groupes E-1 à E-28

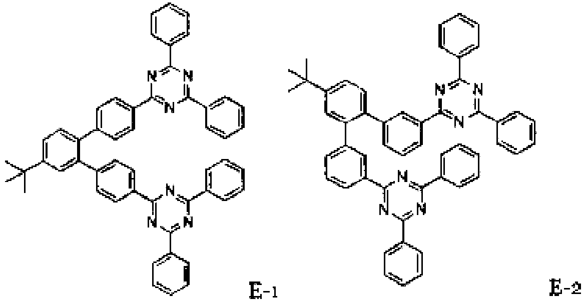

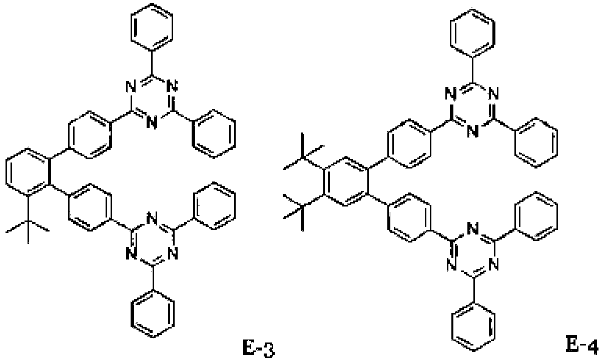

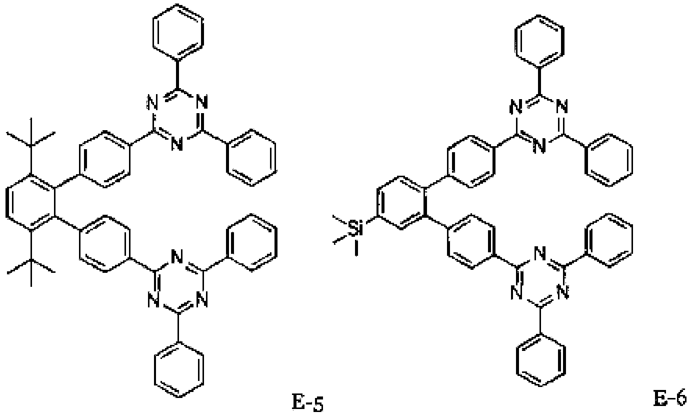

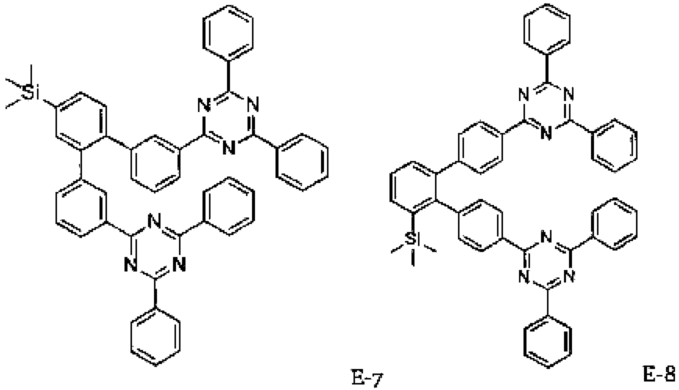

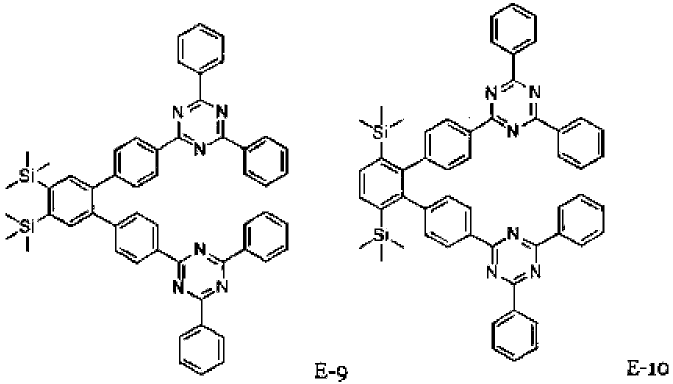

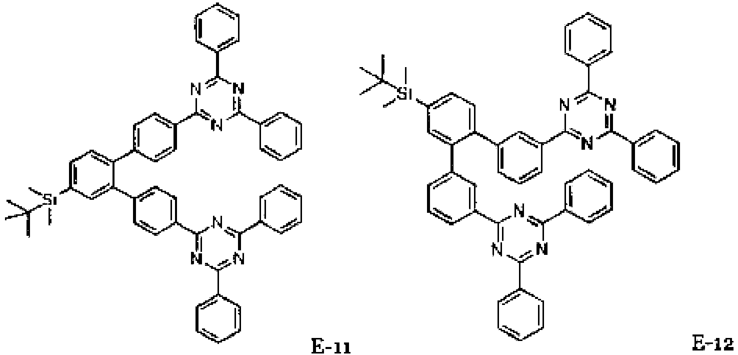

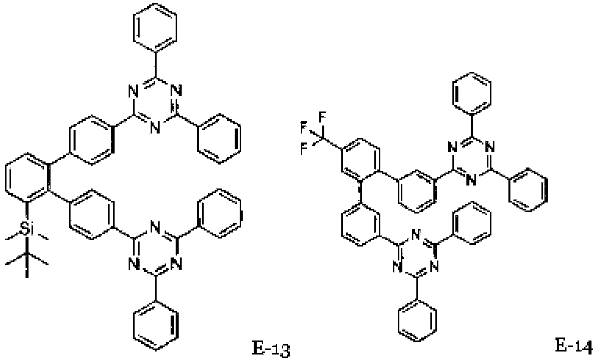

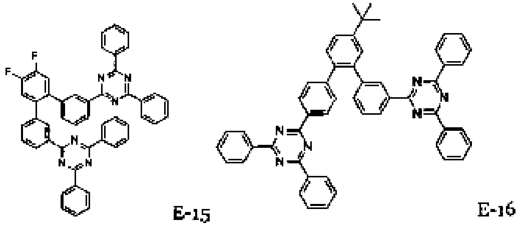

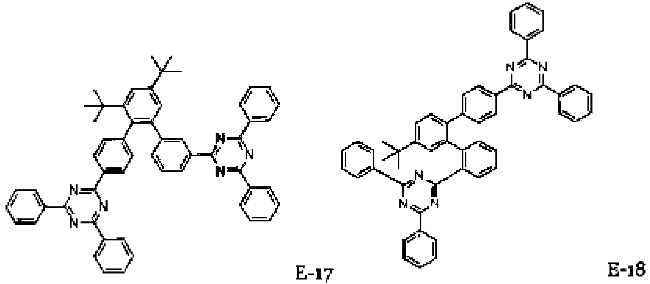

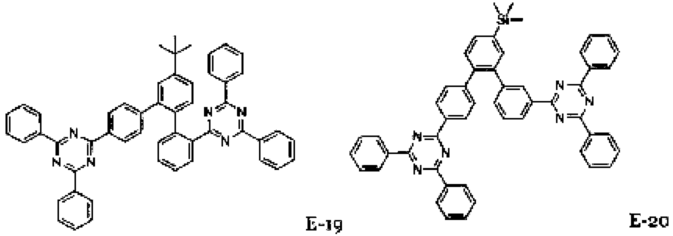

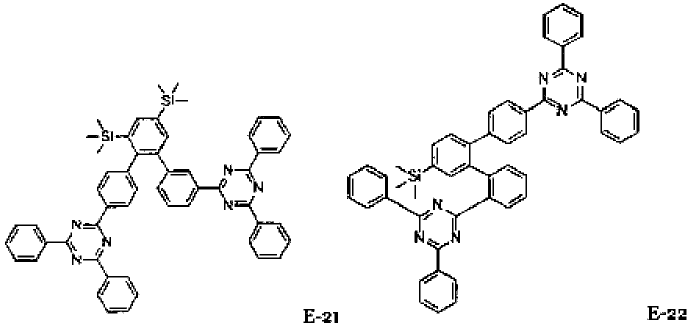

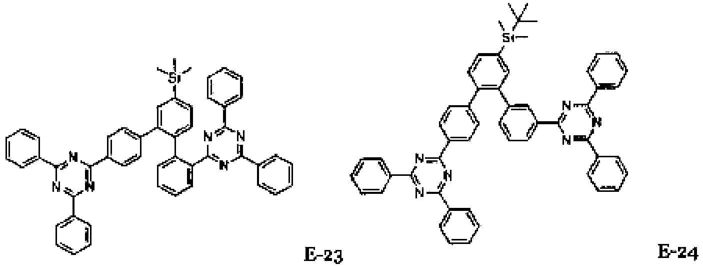

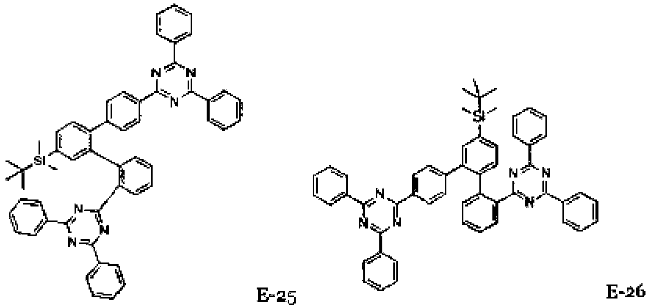

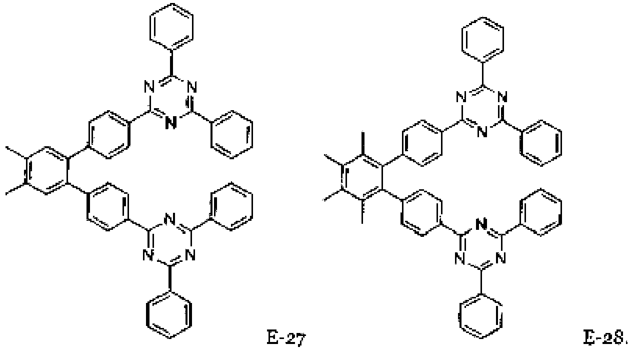

8. Diode électroluminescente organique comprenant un substrat (110), une anode (120), une cathode (190), une couche d'émission (125) et une première couche de transport d'électrons (160), dans laquelle
- la première couche de transport d'électrons est disposée entre la couche d'émission et la cathode ; et
- la première couche de transport d'électrons comprend le composé de formule (I) selon l'une quelconque des revendications précédentes.

9. Diode électroluminescente organique selon la revendication 8, dans laquelle la première couche de transport d'électrons comprend en outre un dopant de type n.

10. Diode électroluminescente organique selon la revendication 8 ou la revendication 9, dans laquelle la diode électroluminescente organique comprend en outre une couche auxiliaire de transport d'électrons et la couche auxiliaire de transport d'électrons est disposée entre la couche d'émission et la première couche de transport d'électrons.

11. Diode électroluminescente organique selon la revendication 10, dans laquelle la première couche de transport d'électrons est en contact entre la couche auxiliaire de transport d'électrons et la couche d'injection d'électrons.

12. Diode électroluminescente organique selon l'une quelconque des revendications 8 à 11, dans laquelle la diode électroluminescente organique comprend en outre une couche d'injection d'électrons et la couche d'injection d'électrons est disposée entre la première couche de transport d'électrons et la cathode.

13. Diode électroluminescente organique selon l'une quelconque des revendications 8 à 12, dans laquelle la diode électroluminescente organique est une diode électroluminescente organique à pile unique ou une diode électroluminescente organique à piles multiples.

14. Dispositif d'affichage comprenant la diode électroluminescente organique selon l'une quelconque des revendications 8 à 13.

15. Dispositif d'affichage selon la revendication 14, dans lequel le dispositif d'affichage est un téléviseur, une tablette ou un téléphone mobile.
